# EUROPEAN PATENT SPECIFICATION

(11) **EP 4 582 025 B1**
(45) Date of publication and mention of the grant of the patent: **12.08.2026**
(21) Application number: 23859938.5
(22) Date of filing: 31.07.2023
(51) Int. Cl.: A61B 6/00, A61B 6/50, G06T 7/11

(54) **INFORMATION PROCESSING DEVICE, INFORMATION PROCESSING METHOD, AND PROGRAM**
INFORMATIONSVERARBEITUNGSVORRICHTUNG, INFORMATIONSVERARBEITUNGSVERFAHREN UND PROGRAMM
DISPOSITIF DE TRAITEMENT D'INFORMATIONS, PROCÉDÉ DE TRAITEMENT D'INFORMATIONS ET PROGRAMME

(30) Priority: 02.09.2022 JP 2022140182
(43) Date of publication of application: 09.07.2025
(73) Proprietor: FUJIFILM Corporation, Tokyo 106-8620 (JP)
(72) Inventor: MACHII, Yusuke, Ashigarakami-gun, Kanagawa 258-8538 (JP)
(74) Representative: Dehns Germany Partnerschaft mbB
(86) International application number: PCT/JP2023/028032
(87) International publication number: WO 2024/048169

(56) References cited:
- WO-A1-2019/017442
- WO-A1-2022/065318
- CN-A- 113 288 186
- JP-A- 2002 521 896
- JP-A- 2020 010 805
- JP-A- 2020 116 283
- JP-A- H04 156 789

## Description

### BACKGROUND OF THE INVENTION

### 1. Field of the Invention

The present disclosure relates to an information processing apparatus, an information processing method, and a program.

### 2. Description of the Related Art

Contrast-enhanced mammography, which acquires a low-energy image and a high-energy image by performing imaging by irradiating a breast in which a contrast agent is injected with radiation having different energies and generates a difference image representing a difference between the low-energy image and the high-energy image to generate an image in which a lesion or the like is contrast-enhanced, is known. In recent years, since the contrast-enhanced mammography has been included in a comprehensive guideline for breast cancer image diagnosis called a breast imaging reporting and data system (BI-RADS), there is a high possibility that the contrast-enhanced mammography will be widely used as a standard diagnosis method.

However, it is difficult to perform the interpretation of the image obtained by the contrast-enhanced mammography. One of the reasons for the difficulty is an effect of background mammary gland parenchymal enhancement (BPE) due to the contrast agent. The BPE represents a level of enhancement of a normal structure of a mammary gland via the contrast agent, and the visibility of the enhanced lesion greatly varies depending on the level of the BPE. As described above, since the difficulty of the interpretation is high in the contrast-enhanced mammography, it is desired to support even a doctor who is not accustomed to the interpretation so that standard interpretation can be performed.

As a technology related to supporting the interpretation of the image in mammography, for example, in Richa Agarwal, et al., 'Deep learning for mass detection in Full Field Digital Mammograms', [online]; Computers in Biology and Medicine 121 (2020) 103774, [retrieved on 2022-08-16]. Retrieved from the Internet: <URL: https://www.sciencedirect.com/science/article/pii/S001048252030144X>., it is proposed to detect a lesion such as breast cancer by using a Faster Region-based Convolutional Neural Network (R-CNN).

WO 2022/065318 A1 discloses an image processing apparatus, image processing method, and image processing program.

### SUMMARY OF THE INVENTION

In the technology described in Richa Agarwal, et al., 'Deep learning for mass detection in Full Field Digital Mammograms', [online]; Computers in Biology and Medicine 121 (2020) 103774, [retrieved on 2022-08-16]. Retrieved from the Internet: <URL: https://www.sciencedirect.com/science/article/pii/S001048252030144X>., extraction of a lesion candidate region and lesion determination are performed based on the same image. For example, since the above-described difference image is contrast-enhanced in a region with a high possibility of the lesion, it is possible to easily detect the lesion candidate region. However, since the difference image has little information on the structure such as the mammary gland, it is difficult to accurately determine whether or not the lesion candidate is the lesion. Therefore, in the related art, there is a trade-off in that the lesion candidate region can be accurately extracted, but the accuracy of the lesion determination is low, and it is not possible to sufficiently support the interpretation of the image.

An object of the present disclosed technology is to provide an information processing apparatus, an information processing method, and a program capable of improving support for interpretation of an image generated by contrast-enhanced imaging.

In order to achieve the above-described object, in a first aspect of the present disclosure there is provided an information processing apparatus as claimed in claim 1.

It is preferable that the processor is configured to: determine whether or not the lesion candidate is the lesion by inputting the patch image to a machine learned model.

It is preferable that the processor is configured to: cut out respective regions corresponding to the same lesion candidate region from at least any one of the low-energy image or the high-energy image and the difference image, as the patch images, and input the cutout regions to the machine learned model.

It is preferable that the subject is a breast, and the processor is configured to: determine an enhancement level of background mammary gland parenchyma of the breast based on the difference image and adjust a parameter for determining a condition under which the machine learned model extracts the lesion candidate region, based on the enhancement level.

It is preferable that the electromagnetic waves are radiation.

It is preferable that the subject is left and right breasts, the low-energy image includes a first low-energy image and a second low-energy image that are captured by irradiating each of the left and right breasts with radiation having the first energy, the high-energy image includes a first high-energy image and a second high-energy image that are captured by irradiating each of the left and right breasts with radiation having the second energy, and the difference image includes a first difference image representing a difference between the first low-energy image and the first high-energy image and a second difference image representing a difference between the second low-energy image and the second high-energy image.

It is preferable that the processor is configured to: extract the lesion candidate region from the first difference image by inputting the first difference image to a first machine learned model; and extract the lesion candidate region from the second difference image by inputting the second difference image to a second machine learned model.

It is preferable that the first machine learned model includes a first pre-stage operation block and a first post-stage operation block, the second machine learned model includes a second pre-stage operation block and a second post-stage operation block, and the processor is configured to: extract a first feature value by inputting the first difference image to the first pre-stage operation block; extract a second feature value by inputting the second difference image to the second pre-stage operation block; extract the lesion candidate region from the first difference image by combining the second feature value with the first feature value and inputting the combined feature value to the first post-stage operation block; and extract the lesion candidate region from the second difference image by combining the first feature value with the second feature value and inputting the combined feature value to the second post-stage operation block.

It is preferable that the processor is configured to: combine the first feature value and the second feature value in a channel direction.

It is preferable that the processor is configured to: combine the first feature value and the second feature value via a cross attention mechanism that generates a weight map representing a degree of relevance between the first feature value and the second feature value and that performs weighting on the first feature value and the second feature value based on the generated weight map.

It is preferable that the processor is configured to: determine a first enhancement level of background mammary gland parenchyma based on the first difference image; determine a second enhancement level of the background mammary gland parenchyma based on the second difference image; determine symmetry of enhancement regions of the background mammary gland parenchyma related to the left and right breasts based on the first difference image and the second difference image; adjust a parameter for determining a condition under which the first machine learned model extracts the lesion candidate region, based on the first enhancement level and the symmetry; and adjust a parameter for determining a condition under which the second machine learned model extracts the lesion candidate region, based on the second enhancement level and the symmetry.

It is preferable that the machine learned model is generated by training a machine learning model using training data including an input image and ground-truth data and an augmented image in which a contrast between a lesion region and a non-lesion region in the input image is changed.

In a second aspect of the present disclosure there is provided an information processing method as claimed in claim 13.

In a third aspect of the present disclosure there is provided a program causing a computer to execute a process as claimed in claim 14.

According to the present disclosed technology, it is possible to provide the information processing apparatus, the information processing method, and the program capable of improving the support for the interpretation of the image generated by the contrast-enhanced imaging.

### BRIEF DESCRIPTION OF THE DRAWINGS

Fig. 1 is a diagram showing an example of an overall configuration of a radiation image capturing system according to a first embodiment.
Fig. 2 is a block diagram showing an example of a configuration of an information processing apparatus.
Fig. 3 is a block diagram showing an example of functions implemented by a control unit of the information processing apparatus.
Fig. 4 is a flowchart schematically showing a flow of contrast-enhanced imaging processing.
Fig. 5 is a flowchart schematically showing a flow of detection processing.
Fig. 6 is a diagram schematically showing lesion candidate region extraction processing via a lesion candidate region extraction processing unit.
Fig. 7 is a diagram schematically showing lesion determination processing via a lesion determination processing unit.
Fig. 8 is a diagram conceptually showing an example of a configuration of a machine learned model.
Fig. 9 is a diagram schematically showing lesion candidate region extraction processing according to a first modification example.
Fig. 10 is a diagram schematically showing lesion determination processing according to a second modification example.
Fig. 11 is a diagram schematically showing lesion candidate region extraction processing according to a third modification example.
Fig. 12 is a diagram showing an example of a determination result of a BPE level.
Fig. 13 is a diagram schematically showing lesion candidate region extraction processing according to a fourth modification example.
Fig. 14 is a diagram schematically showing a modification example of combination processing.
Fig. 15 is a diagram schematically showing combination processing using a cross attention mechanism.
Fig. 16 is a diagram schematically showing lesion candidate region extraction processing according to a fifth modification example.
Fig. 17 is a diagram conceptually showing an example of machine learning processing of a machine learning model.
Fig. 18 is a diagram conceptually showing data augmentation processing.
Fig. 19 is a diagram schematically showing lesion candidate region extraction processing via a lesion candidate region extraction processing unit according to a second embodiment.
Fig. 20 is a diagram schematically showing lesion determination processing via a lesion determination processing unit according to the second embodiment.
Fig. 21 is a block diagram showing a configuration of an information processing apparatus according to a modification example of the second embodiment.
Fig. 22 is a block diagram showing a configuration of an information processing apparatus according to another modification example of the second embodiment.

### DESCRIPTION OF THE PREFERRED EMBODIMENTS

Hereinafter, an embodiment of the present disclosure will be described in detail with reference to the accompanying drawings.

### [First Embodiment]

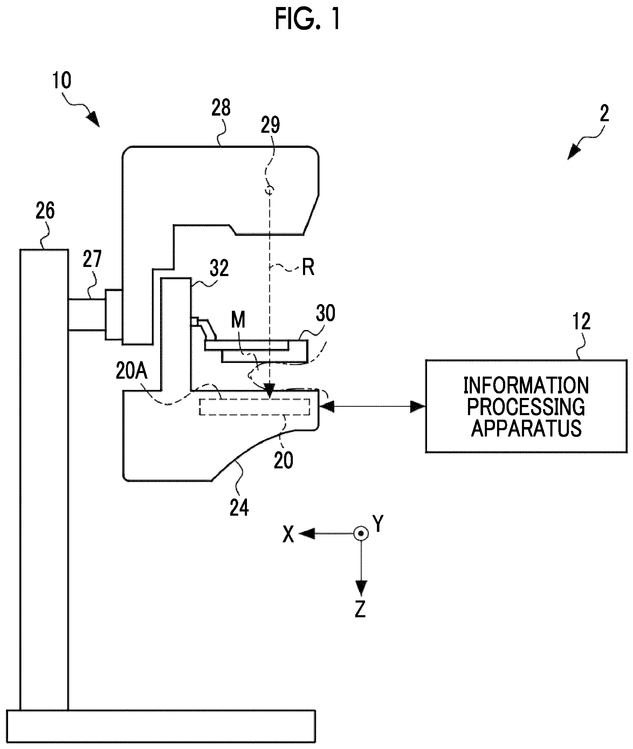

Fig. 1 shows an example of an overall configuration of a radiation image capturing system 2 according to the first embodiment. The radiation image capturing system 2 comprises a mammography apparatus 10 and an information processing apparatus 12. The information processing apparatus 12 is connected to a radiology information system (RIS), a picture archiving and communication system (PACS), and the like (none of which is shown) via a network or the like.

Fig. 1 shows an example of an appearance of the mammography apparatus 10. It should be noted that Fig. 1 shows an example of the appearance in a case in which the mammography apparatus 10 is seen from a left side of a person under an examination.

The mammography apparatus 10 is a radiography apparatus that operates under the control of the information processing apparatus 12 and that irradiates a breast M of a person under an examination, as a subject, with radiation R (for example, X-rays) from a radiation source 29 to capture a radiation image of the breast M. It should be noted that the radiation R is an example of "electromagnetic waves" according to the present disclosed technology.

As shown in Fig. 1, the mammography apparatus 10 comprises an imaging table 24, a base 26, an arm part 28, and a compression unit 32. A radiation detector 20 is disposed inside the imaging table 24. As shown in Fig. 1, in a case in which imaging is performed, in the mammography apparatus 10, the breast M of the person under an examination is positioned on the imaging table 24 by a user, such as a radiologist.

The radiation detector 20 detects the radiation R passing through the breast M as the subject. Specifically, the radiation detector 20 detects the radiation R passing through the breast M of the person under an examination, entering into the imaging table 24, and reaching a detection surface 20A of the radiation detector 20, and generates a radiation image based on the detected radiation R. The radiation detector 20 outputs image data representing the generated radiation image. Hereinafter, the series of operations of irradiating the breast with the radiation R from the radiation source 29 to generate the radiation image via the radiation detector 20 may be referred to as "imaging". The radiation detector 20 may be an indirect conversion type radiation detector that converts the radiation R into light beams and converts the converted light beams into charges, or may be a direct conversion type radiation detector that directly converts the radiation R into charges.

Hereinafter, two directions orthogonal to each other and parallel to the detection surface 20A will be referred to as an X direction and a Y direction. In addition, a direction orthogonal to the X direction and the Y direction will be referred to as a Z direction.

A compression plate 30 that is used for compressing the breast M in a case of performing the imaging is attached to the compression unit 32. The compression plate 30 is moved in a direction approaching or in a direction spaced away from the imaging table 24 by a compression plate drive unit (not shown) provided in the compression unit 32. The compression plate 30 is moved in a direction approaching the imaging table 24 to compress the breast M with the imaging table 24.

The arm part 28 can be rotated with respect to the base 26 by a shaft part 27. The shaft part 27 is fixed to the base 26, and the shaft part 27 and the arm part 28 are rotated integrally. Gears are provided in each of the shaft part 27 and the compression unit 32 of the imaging table 24, and the gears are switched between an engaged state and a non-engaged state, so that a state in which the compression unit 32 of the imaging table 24 and the shaft part 27 are connected to each other and are rotated integrally and a state in which the shaft part 27 is separated from the imaging table 24 and idles can be switched. The elements for switching between transmission and non-transmission of power of the shaft part 27 are not limited to the gears, and various mechanical elements can be used. The arm part 28 and the imaging table 24 can be separately rotated relative to the base 26 with the shaft part 27 as a rotation axis.

The mammography apparatus 10 can perform the imaging on each of the left and right breasts M from a plurality of directions by rotating the arm part 28. For example, it is possible to perform cranio-caudal (CC) imaging and medio-lateral oblique (MLO) imaging.

The radiation image capturing system 2 can perform "contrast-enhanced imaging" in which the imaging is performed in a state in which a contrast agent is injected in the breast M. Specifically, the radiation image capturing system 2 has a contrast enhanced digital mammography (CEDM) function of performing contrast enhancement via energy subtraction.

In the contrast-enhanced imaging, a low-energy image and a high-energy image are acquired by performing the imaging by irradiating the breast M, in which the contrast agent is injected, with the radiation R having different energies. In the present disclosure, a radiation image captured by the radiation R having a first energy will be referred to as a "low-energy image", and a radiation image captured by the radiation R having a second energy higher than the first energy will be referred to as a "high-energy image". Hereinafter, in a case in which the low-energy image and the high-energy image are not distinguished from each other, the low-energy image and the high-energy image will be simply referred to as a radiation image.

In the contrast-enhanced imaging, for example, an iodine contrast agent having a k absorption edge of 32 keV is used as the contrast agent. In the contrast-enhanced imaging in a case in which the iodine contrast agent is used, the first energy need only be set to be lower than the k absorption edge, and the second energy need only be set to be higher than the k absorption edge.

The contrast agent and the body tissue such as the mammary gland are different in absorption characteristics of the radiation R. Therefore, the high-energy image clearly shows the contrast agent in addition to the body tissue such as the mammary gland and the fat. On the other hand, in the low-energy image, the body tissue is clearly shown, but the contrast agent is hardly shown. Therefore, by taking a difference between the low-energy image and the high-energy image, it is possible to generate a difference image in which the mammary gland structure is erased and a lesion or the like stained with the contrast agent is enhanced. The lesion consists of, for example, new cells and is easily stained with the contrast agent.

The mammography apparatus 10 and the information processing apparatus 12 are connected by wired communication or wireless communication. The radiation image generated by the radiation detector 20 in the mammography apparatus 10 is output to the information processing apparatus 12 by wired communication or wireless communication via a communication interface (I/F) (not shown).

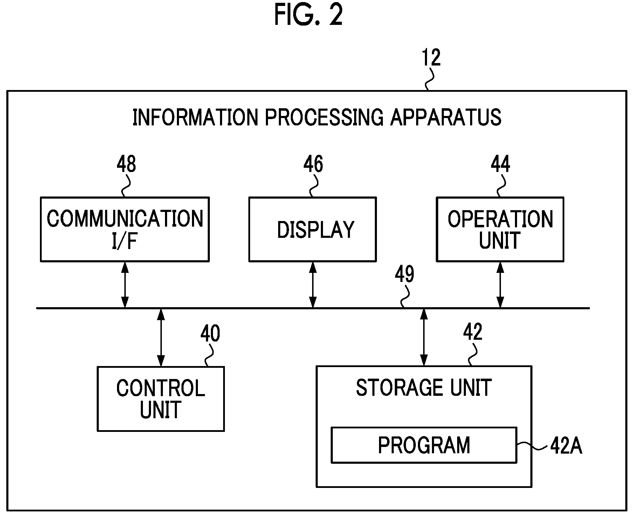

Fig. 2 shows an example of the configuration of the information processing apparatus 12. The information processing apparatus 12 comprises a control unit 40, a storage unit 42, an operation unit 44, a display 46, and a communication I/F 48. The control unit 40, the storage unit 42, the operation unit 44, the display 46, and the communication I/F 48 are connected to each other via a bus 49 such that various kinds of information can be exchanged.

The control unit 40 controls an overall operation of the radiation image capturing system 2. The control unit 40 is configured by, for example, a computer comprising a central processing unit (CPU), a read only memory (ROM), and a random access memory (RAM).

The storage unit 42 stores information related to radiography, the radiation image acquired from the mammography apparatus 10, and the like. In addition, the storage unit 42 stores a program 42A for the control unit 40 to perform various kinds of information processing described later and data for constructing various kinds of machine learned models described later. The storage unit 42 is, for example, a nonvolatile storage device such as a hard disk drive (HDD) or a solid state drive (SSD).

The operation unit 44 includes input devices such as various buttons, switches, a touch panel, a touch pen, and a mouse, which are operated by the user. The display 46 displays information related to imaging, a radiation image obtained by imaging, a determination result obtained by lesion determination processing described later, and the like.

The communication I/F 48 performs communication of various kinds of data, such as information related to the radiography and the radiation image, with the mammography apparatus 10, the RIS, the PACS, and the like via wired communication or wireless communication.

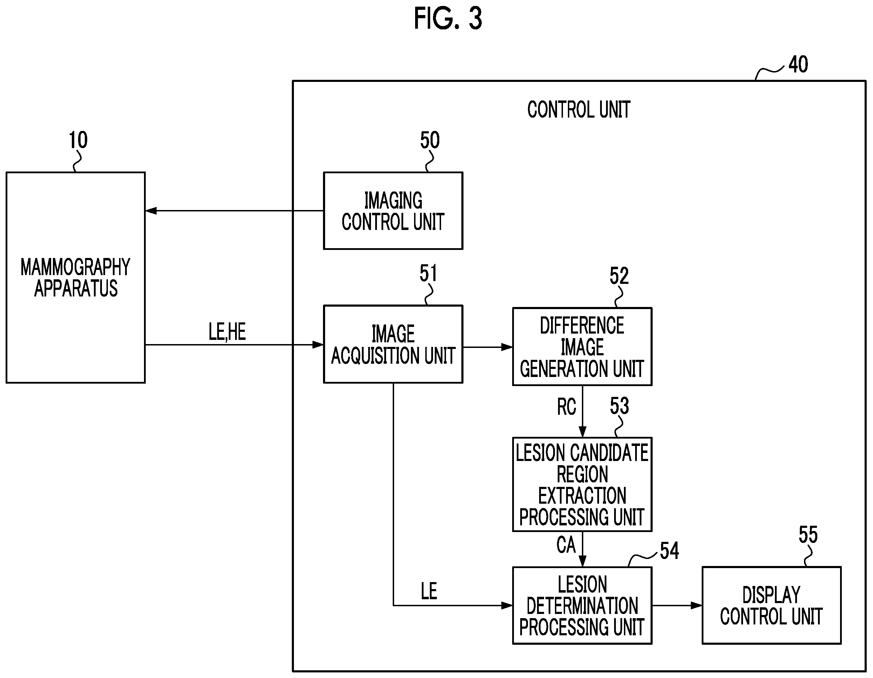

Fig. 3 shows an example of functions implemented by the control unit 40 of the information processing apparatus 12. The control unit 40 implements various functions by executing the processing based on the program 42A stored in the storage unit 42. The control unit 40 functions as an imaging control unit 50, an image acquisition unit 51, a difference image generation unit 52, a lesion candidate region extraction processing unit 53, a lesion determination processing unit 54, and a display control unit 55.

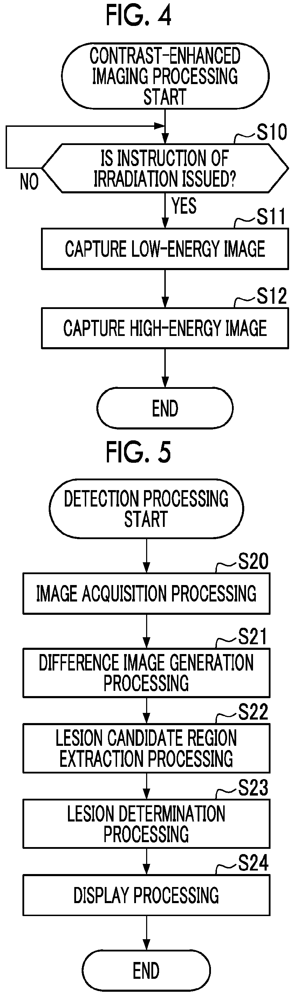

Fig. 4 schematically shows a flow of contrast-enhanced imaging processing. Processing via the imaging control unit 50 will be described with reference to Fig. 4.

First, before the imaging via the mammography apparatus 10 is started, the user, such as the radiologist, injects the contrast agent into the breast M of the person under an examination, positions the breast M in which the contrast agent is injected on the imaging table 24, and compresses the breast M with the compression plate 30.

In step S10, the imaging control unit 50 determines whether or not an instruction of the irradiation with the radiation R is received. In a case in which the instruction of the irradiation is received, the imaging control unit 50 outputs, in step S11, an instruction of the irradiation with the radiation R having the first energy to the mammography apparatus 10. In the mammography apparatus 10, a low-energy image LE is captured by emitting the first energy radiation R toward the breast M.

In next step S12, the imaging control unit 50 outputs an instruction of the irradiation with the radiation R having the second energy to the mammography apparatus 10. In the mammography apparatus 10, a high-energy image HE is captured by emitting the radiation R having the second energy toward the breast M. It should be noted that the high-energy image HE may be captured earlier than the low-energy image LE.

In a case in which the capturing of the low-energy image LE and the high-energy image HE of the breast M ends, the user releases the compression of the breast M for which the imaging ends.

Fig. 5 schematically shows a flow of detection processing. Processing via the imaging control unit 50, the image acquisition unit 51, the difference image generation unit 52, the lesion candidate region extraction processing unit 53, the lesion determination processing unit 54, and the display control unit 55 will be described with reference to Fig. 5.

In step S20, the image acquisition unit 51 acquires the low-energy image LE and the high-energy image HE captured by the above-described contrast-enhanced imaging processing.

In next step S21, the difference image generation unit 52 generates a difference image RC representing a difference between the low-energy image LE and the high-energy image HE. For example, the difference image generation unit 52 generates the difference image RC by subtracting an image obtained by multiplying the low-energy image LE by a first weight coefficient from an image obtained by multiplying the high-energy image HE by a second weight coefficient for each corresponding pixel.

In next step S22, the lesion candidate region extraction processing unit 53 performs lesion candidate region extraction processing of extracting a lesion candidate region including a lesion candidate from the difference image RC.

In next step S23, the lesion determination processing unit 54 performs lesion determination processing of determining whether or not the lesion candidate included in the lesion candidate region is a lesion (that is, whether the lesion is benign or malignant) by using the low-energy image LE.

In next step S24, the display control unit 55 displays the determination result of the lesion obtained by the lesion determination processing on the display 46. The display control unit 55 may display the low-energy image LE on the display 46 along with the determination result of the lesion. In addition, the display control unit 55 may display the difference image RC or the high-energy image HE on the display 46 along with the determination result of the lesion.

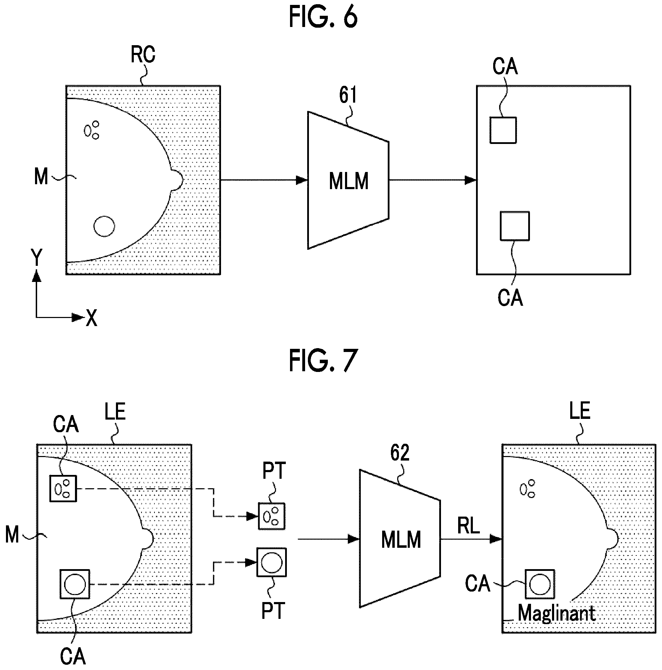

Fig. 6 schematically shows the lesion candidate region extraction processing via the lesion candidate region extraction processing unit 53. The lesion candidate region extraction processing unit 53 extracts a lesion candidate region CA by inputting the difference image RC to a first machine learned model (MLM) 60 that functions as a lesion candidate region extraction unit. The MLM 61 is configured by a convolutional neural network (CNN). For example, the MLM 61 is configured by an R-CNN that detects an object from an image, or the like.

For example, the MLM 61 performs three kinds of processing of region proposal processing, feature value extraction processing, and class classification processing, to extract the lesion candidate region CA including the lesion candidate estimated as the lesion from the difference image RC.

Specifically, in the region proposal processing, a region in which the object is likely to be included is detected from the difference image RC as a region of interest (hereinafter, referred to as an ROI). In the feature value extraction processing, the feature value is extracted by performing convolution processing or the like on the ROI. In the class classification processing, the object included in the ROI is classified based on the extracted feature value. The ROI including the object classified into the class as the lesion candidate is extracted as the lesion candidate region CA. In the example shown in Fig. 6, two lesion candidate regions CA are extracted from the difference image RC.

Fig. 7 schematically shows lesion determination processing via the lesion determination processing unit 54. The lesion determination processing unit 54 cuts out a region corresponding to the lesion candidate region CA from the low-energy image LE, as a patch image PT, and inputs the cutout patch image PT to an MLM 62 functioning as a lesion determination unit, thereby determining whether or not the lesion candidate included in the patch image PT is the lesion. The MLM 62 outputs a determination result RL of the lesion determination, for each input patch image PT.

In the example shown in Fig. 7, the lesion determination is performed for each of two patch images PT by sequentially inputting the two patch images PT cut out from the low-energy image LE to the MLM 62. One of the two patch images PT is determined to be malignant.

For example, the display control unit 55 displays the difference image RC in which the lesion candidate region CA corresponding to the patch image PT determined to be malignant by the lesion determination is indicated by a bounding box, on the display 46. In addition, for example, the display control unit 55 displays a label (Malignant) indicating that the lesion candidate included in the lesion candidate region CA is malignant, on the display 46. It should be noted that the display control unit 55 may indicate the lesion not only by using the bounding box, but also by using a marker such as an arrow, a highlight display using a color, or the like.

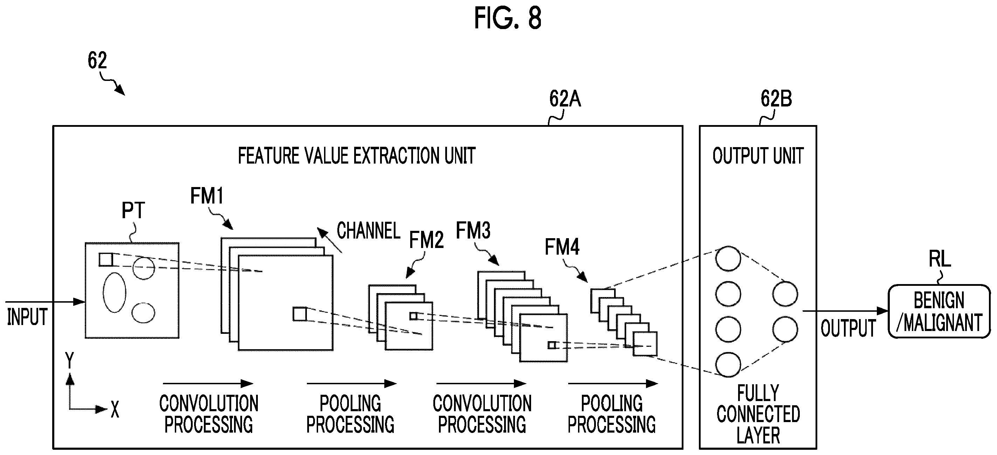

Fig. 8 conceptually shows an example of the configuration of the MLM 62. The MLM 62 includes a feature value extraction unit 62A and an output unit 62B. The feature value extraction unit 62A is an intermediate layer including a plurality of convolutional layers and a plurality of pooling layers. In the present embodiment, the output unit 62B is an output layer including a fully connected layer.

The patch image PT is input to the feature value extraction unit 62A. The feature value extraction unit 62A extracts a feature value by executing convolution processing and pooling processing on the input patch image PT. In the example shown in Fig. 8, the feature value extraction unit 62A generates a three-channel feature map FM1 by executing the convolution processing on the patch image PT, and generates a three-channel feature map FM2 having a reduced size by executing the pooling processing on the generated feature map FM1. Then, the feature value extraction unit 62A generates a six-channel feature map FM3 by executing the convolution processing on the feature map FM2, and generates a six-channel feature map FM4 having a reduced size by executing the pooling processing on the generated feature map FM3. The number of channels is determined by the number of filters used in a case in which the convolution processing is performed.

The output unit 62B performs the class classification based on the feature value extracted by the feature value extraction unit 62A, and outputs a result of the class classification as the determination result RL. In the example shown in Fig. 8, the feature map FM4 is input from the feature value extraction unit 62A to the output unit 62B. The output unit 62B performs the class classification based on the feature map FM4. In the present embodiment, the output unit 62B classifies the lesion candidate into two classes of benign and malignant. That is, the determination result RL indicates whether the lesion candidate is benign or malignant.

It should be noted that the number of convolutional layers, the number of pooling layers, the number of filters used for the convolution processing, and the like included in the feature value extraction unit 62A can be changed as appropriate.

As described above, in the present embodiment, the lesion candidate region CA is extracted from the difference image RC, the region corresponding to the lesion candidate region CA is cut out from the low-energy image LE, as the patch image PT, and the lesion determination processing is performed based on the cutout patch image PT. Since the low-energy image LE includes more information on the structure, such as the mammary gland, than the difference image RC, it is possible to accurately perform the lesion determination. Therefore, according to the present embodiment, since the lesion candidate region can be accurately extracted and whether or not the lesion candidate is the lesion can be accurately determined, the support for the interpretation of the image generated by the contrast-enhanced imaging is improved.

It should be noted that, in the present embodiment, the lesion determination processing unit 54 cuts out the patch image PT from the low-energy image LE and inputs the cutout patch image PT to the MLM 62, but the lesion determination processing unit 54 may cut out the patch image PT from the high-energy image HE instead of the low-energy image LE and input the cutout patch image PT to the MLM 62. In addition, the lesion determination processing unit 54 may cut out respective regions corresponding to the same lesion candidate region CA from the low-energy image LE and the high-energy image HE as the patch images PT and input the patch images PT to the MLM 62. In this case, it is preferable that the lesion determination processing unit 54 inputs the patch image PT cut out from the low-energy image LE and the patch image PT cut out from the high-energy image HE to the MLM 62 by combining the patch image PT and the patch image PT in a channel direction for each lesion candidate region CA. The present disclosed technology is characterized in that the lesion determination is performed by cutting out the region corresponding to the lesion candidate region CA from at least any one of the low-energy image LE or the high-energy image HE, as the patch image PT.

Hereinafter, various modification examples of the first embodiment will be described.

### [First Modification Example]

The first modification example is different from the above-described embodiment only in the lesion candidate region extraction processing via the lesion candidate region extraction processing unit 53. In the present modification example, the lesion candidate region extraction processing unit 53 extracts the lesion candidate region CA based on a result of segmentation performed by the MLM 61 based on the difference image RC.

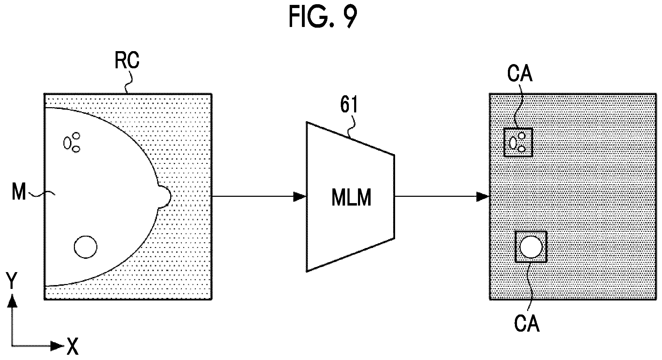

Fig. 9 schematically shows the lesion candidate region extraction processing according to the first modification example. In the present modification example, the MLM 61 is configured by, for example, a U-net, which is one kind of a CNN, and performs class classification of each pixel of the difference image RC. The MLM 61 specifies a pixel region belonging to a lesion candidate class and outputs the specified pixel region. The lesion candidate region extraction processing unit 53 extracts the lesion candidate region CA by setting a rectangular bounding box so as to surround the pixel region specified by the MLM 61. Other processing is the same as the processing according to the above-described embodiment.

### [Second Modification Example]

The second modification example is different from the above-described embodiment only in the lesion determination processing via the lesion determination processing unit 54. In the present modification example, the lesion determination processing unit 54 cuts out the patch image PT from the low-energy image LE and the difference image RC, to perform the lesion determination.

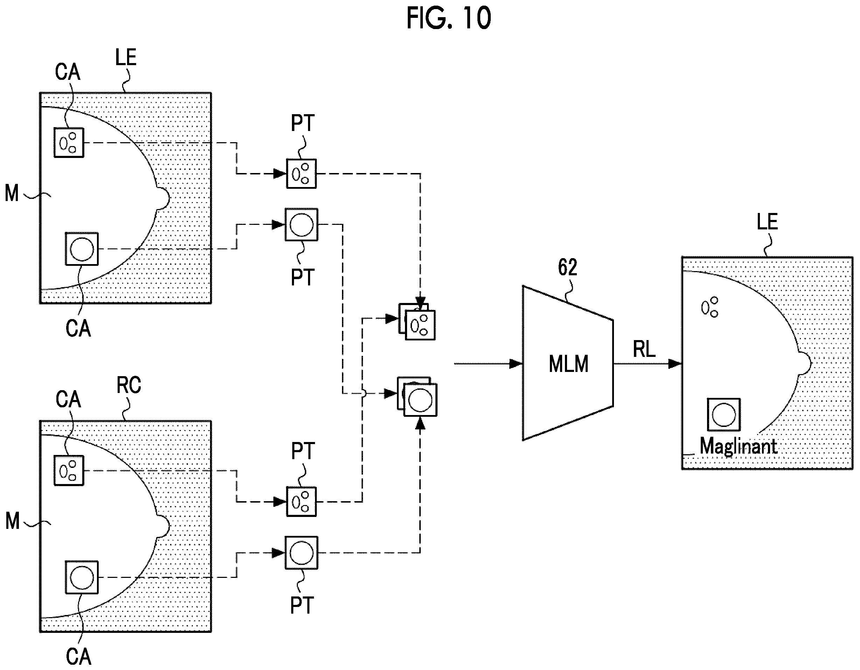

Fig. 10 schematically shows the lesion determination processing according to the second modification example. In the present modification example, the lesion determination processing unit 54 cuts out respective regions corresponding to the same lesion candidate region CA from the low-energy image LE and the difference image RC, as the patch images PT, and inputs the patch images PT to the MLM 62. The lesion determination processing unit 54 combines the patch image PT cut out from the low-energy image LE and the patch image PT cut out from the difference image RC in the channel direction for each lesion candidate region CA and inputs the combined patch image PT to the MLM 62. Other processing is the same as the processing according to the above-described embodiment.

It should be noted that the lesion determination processing unit 54 may cut out the patch images PT from the high-energy image HE and the difference image RC for each lesion candidate region CA, to perform the lesion determination. In addition, the lesion determination processing unit 54 may cut out the patch images PT from the low-energy image LE, the high-energy image HE, and the difference image RC for each lesion candidate region CA, to perform the lesion determination.

### [Third Modification Example]

The third modification example is different from the above-described embodiment only in the lesion candidate region extraction processing via the lesion candidate region extraction processing unit 53. The present modification example is different from the above-described embodiment in that the lesion candidate region extraction processing unit 53 performs a BPE determination based on the difference image RC and extracts the lesion candidate region CA in consideration of a result of the BPE determination.

The BPE determination refers to determining at least any one of an enhancement level of background mammary gland parenchyma or symmetry of enhancement regions of the background mammary gland parenchyma related to the left and right breasts. Hereinafter, the enhancement level of the background mammary gland parenchyma will be referred to as a "BPE level", the enhancement region of the background mammary gland parenchyma will be referred to as a "BPE region", and the symmetry of the BPE regions related to the left and right breasts will be referred to as "BPE symmetry". The BPE level represents a ratio of the enhanced mammary gland parenchyma to the background mammary gland.

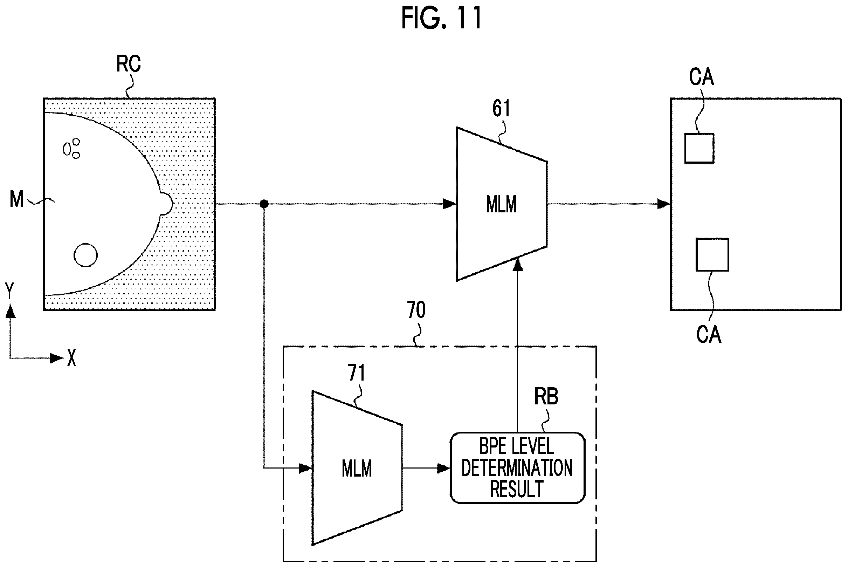

Fig. 11 schematically shows the lesion candidate region extraction processing according to the third modification example. In the present modification example, the lesion candidate region extraction processing unit 53 determines the BPE level via the BPE determination processing unit 70. In the present modification example, the BPE determination processing unit 70 includes an MLM 71 that functions as a BPE level determination unit. The lesion candidate region extraction processing unit 53 inputs the difference image RC to the MLM 61 and the MLM 71. The MLM 71 is configured by a CNN, performs the extraction of the feature value and the class classification based on the difference image RC, and outputs a result of the class classification as a determination result RB.

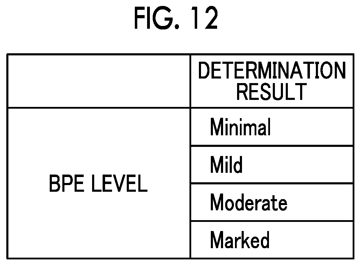

Fig. 12 shows an example of a determination result of the BPE level. As shown in Fig. 12, the determination results of the BPE level are classified into four classes of "Minimal", "Mild", "Moderate", and "Marked". A ratio of the contrast-enhanced mammary gland parenchyma to the background mammary gland is "Minimal" in a case of being less than 25%, "Mild" in a case of being 25% or more and less than 50%, "Moderate" in a case of being 50% or more and less than 75%, and "Marked" in a case of being 75% or more. In "Marked", there is a possibility that the lesion is buried in the background mammary gland and the lesion is invisible.

The MLM 71 outputs a class to which the BPE level belongs among the four classes, as the determination result RB.

As shown in Fig. 11, the lesion candidate region extraction processing unit 53 inputs the determination result RB, which is output from the BPE determination processing unit 70, to the MLM 61. In the present modification example, the lesion candidate region extraction processing unit 53 adjusts a parameter, such as a threshold value, for determining a condition under which the MLM 61 extracts the lesion candidate region CA, based on the determination result RB. For example, the MLM 61 includes a classifier that extracts the ROI as the lesion candidate region CA in a case in which a score (evaluation value) calculated based on the feature value extracted from the ROI is equal to or greater than a threshold value. The lesion candidate region extraction processing unit 53 changes the threshold value of the classifier included in the MLM 61 in accordance with the BPE level represented by the determination result RB. For example, in a case in which the BPE level is high, there is a possibility that the lesion is overlooked because the lesion is buried in the background mammary gland as described above, so that the lesion candidate region extraction processing unit 53 extracts a large number of lesion candidate regions CA by decreasing the threshold value of the classifier.

According to the present modification example, it is possible to perform the lesion candidate region extraction processing having high robustness against the variation in the BPE level, and the support for the interpretation of the image generated by the contrast-enhanced imaging is further improved.

It should be noted that the lesion candidate region extraction processing unit 53 may change the number of lesion candidate regions CA output by the classifier in accordance with the BPE level, instead of the threshold value of the classifier. In this case, the lesion candidate region extraction processing unit 53 increases the number of lesion candidate regions CA output by the classifier as the BPE level is higher.

### [Fourth Modification Example]

The fourth modification example shows an example in which the lesion candidate region extraction processing is performed on the left and right breasts M. In the present modification example, the image acquisition unit 51 acquires the low-energy image LE and the high-energy image HE captured by the contrast-enhanced imaging processing for each of the left and right breasts M. Hereinafter, the low-energy image LE and the high-energy image HE for the left breast M will be referred to as a "first low-energy image LE1" and a "first high-energy image HE1", respectively. In addition, the low-energy image LE and the high-energy image HE for the right breast M will be referred to as a "second low-energy image LE2" and a "second high-energy image HE2", respectively.

In the present modification example, the difference image generation unit 52 generates the difference image RC representing the difference between the low-energy image LE and the high-energy image HE for each of the left and right breasts M. Hereinafter, the difference image RC representing the difference between the first low-energy image LE1 and the first high-energy image HE1 will be referred to as a "first difference image RC1", and the difference image RC representing the difference between the second low-energy image LE2 and the second high-energy image HE2 will be referred to as a "second difference image RC2".

In the present modification example, the lesion candidate region extraction processing unit 53 extracts the lesion candidate region CAfrom the first difference image RC1, and extracts the lesion candidate region CA from the second difference image RC2.

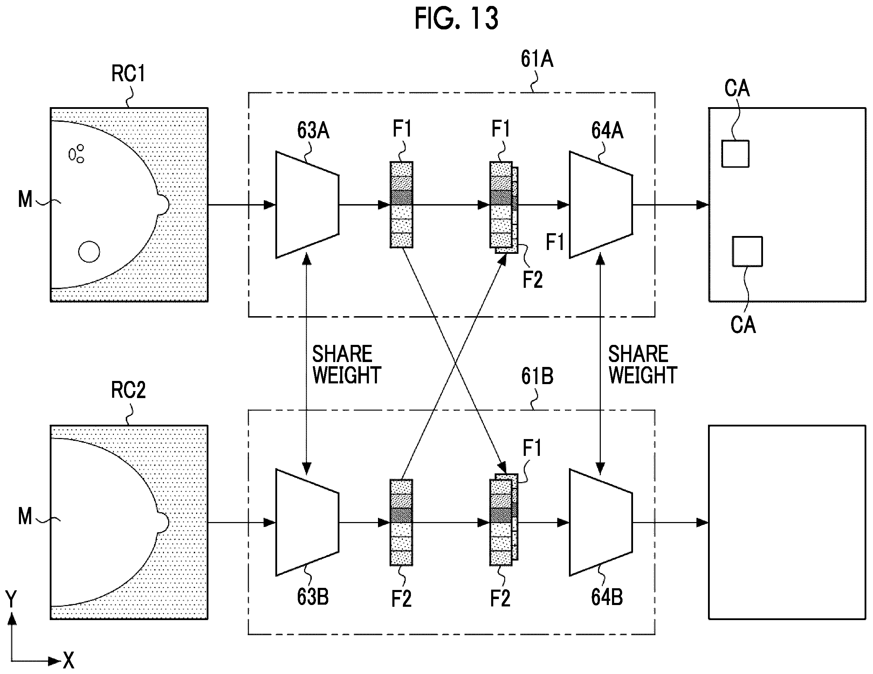

Fig. 13 schematically shows the lesion candidate region extraction processing according to the fourth modification example. The MLM 61A includes a first pre-stage operation block 63A and a first post-stage operation block 64A. The MLM 61B includes a second pre-stage operation block 63B and a second post-stage operation block 64B. The lesion candidate region extraction processing unit 53 inputs the first difference image RC1 to the MLM 61A, and inputs the second difference image RC2 to the MLM 61B.

The first pre-stage operation block 63A outputs the first feature map F1 generated by performing the convolution processing. The second pre-stage operation block 63B outputs the second feature map F2 generated by performing the convolution processing. The lesion candidate region extraction processing unit 53 combines the second feature map F2 with the first feature map F1 to input the combined feature map to the first post-stage operation block 64A, and combines the first feature map F1 with the second feature map F2 to input the combined feature map to the second post-stage operation block 64B. In the present modification example, the lesion candidate region extraction processing unit 53 combines the first feature map F1 and the second feature map F2 in the channel direction. It should be noted that the first feature map F1 is an example of a "first feature value" according to the present disclosed technology, and the second feature map F2 is an example of a "second feature value" according to the present disclosed technology.

The first pre-stage operation block 63A and the second pre-stage operation block 63B have the same configuration, and share the weights of the filters. The first post-stage operation block 64A and the second post-stage operation block 64B have the same configuration, and share the weights of the filters.

The first post-stage operation block 64A extracts the lesion candidate region CA based on the combined first feature map F1 and second feature map F2. Similarly, the second post-stage operation block 64B extracts the lesion candidate region CA based on the combined first feature map F1 and second feature map F2.

In the present modification example, since the first feature map F1 and the second feature map F2 are combined in a cross manner, the machine learning and the lesion candidate region extraction processing can be performed in consideration of the symmetry of the left and right breasts M. For example, a lesion candidate detected from only one of the left breast M or the right breast M is likely to be the lesion. In the present modification example, it is possible to accurately detect the region including the left-right asymmetric structure that is highly likely to be the lesion, as the lesion candidate region CA.

In the present modification example, the lesion determination processing unit 54 cuts out the patch image PT from the first low-energy image LE1 and inputs the cutout patch image PT to the MLM 62, for the lesion candidate region CA extracted from the first difference image RC1. In addition, the lesion determination processing unit 54 cuts out the patch image PT from the second low-energy image LE2 and inputs the cutout patch image PT to the MLM 62, for the lesion candidate region CA extracted from the second difference image RC2. The lesion determination processing according to the present modification example is the same as that in the above-described embodiment, and the same modification as in the second modification example can be made.

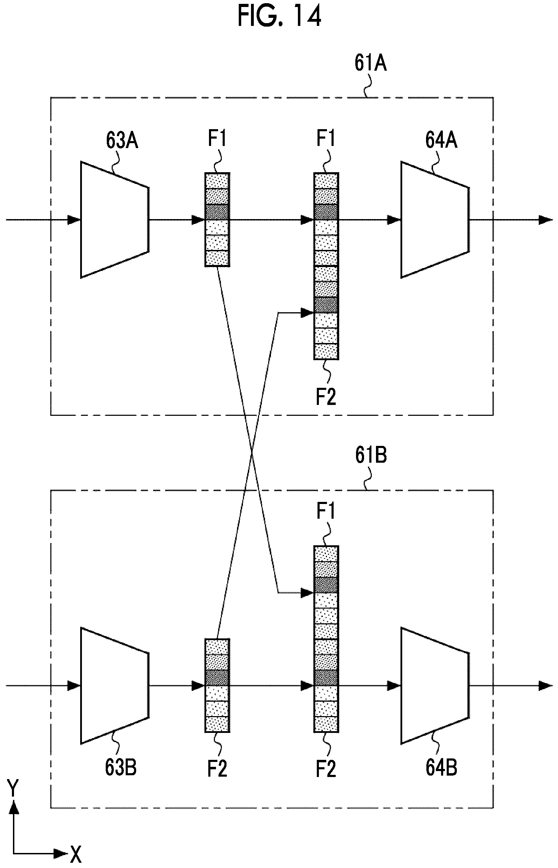

It should be noted that, in the present modification example, although the lesion candidate region extraction processing unit 53 combines the first feature map F1 and the second feature map F2 in the channel direction, as shown in Fig. 14, the first feature map F1 and the second feature map F2 may be combined in a row direction or a column direction (that is, the X direction or the Y direction).

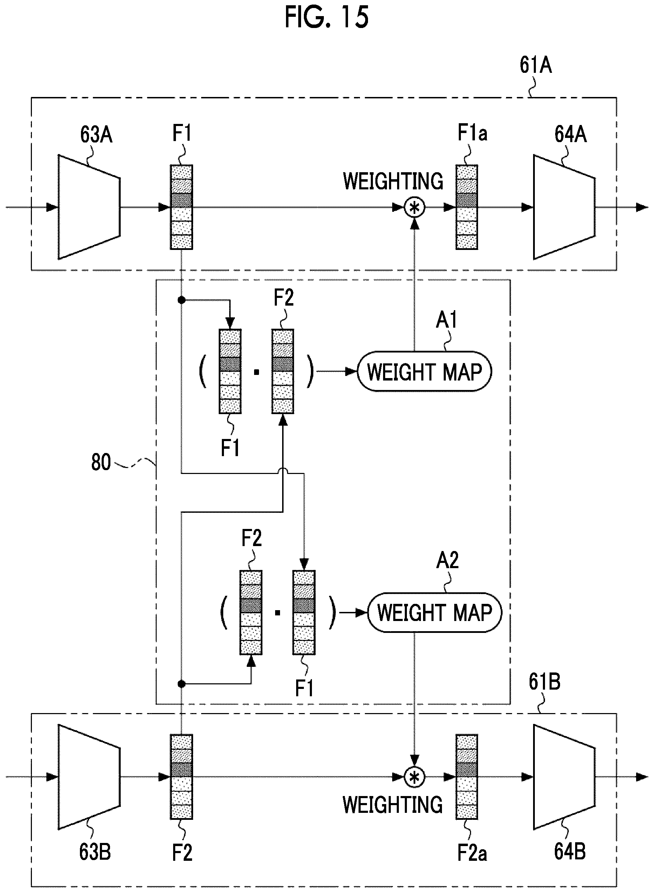

In addition, as shown in Fig. 15, the lesion candidate region extraction processing unit 53 may combine the first feature map F1 and the second feature map F2 via a cross attention mechanism 80. The cross attention mechanism 80 performs a matrix operation of multiplying the first feature map F1 by the second feature map F2 to calculate a weight map A1, and performs a matrix operation of multiplying the second feature map F2 by the first feature map F1 to calculate a weight map A2. Each of the weight map A1 and the weight map A2 represents a degree of relevance between the first feature map F1 and the second feature map F2.

The cross attention mechanism 80 performs weighting of the first feature map F1 based on the weight map A1, and performs weighting of the second feature map F2 based on the weight map A2. A first feature map F1a subjected to the weighting is input to the first post-stage operation block 64A. The second feature map F2a subjected to the weighting is input to the second post-stage operation block 64B.

### [Fifth Modification Example]

The fifth modification example is different from the fourth modification example in that the lesion candidate region extraction processing unit 53 includes a BPE determination processing unit 70A.

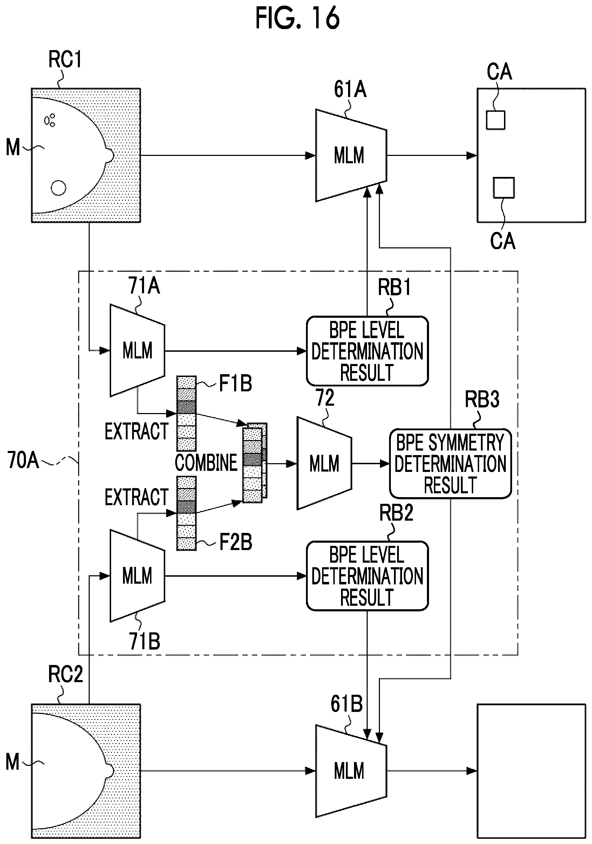

Fig. 16 schematically shows the lesion candidate region extraction processing according to a fifth modification example. In the present modification example, the BPE determination processing unit 70A includes an MLM 71A, an MLM 71B, and an MLM 72. Each of the MLM 71A and the MLM 71B has the same configuration as the MLM 71 (see Fig. 11). The MLM 71A functions as a first BPE level determination unit. The MLM 71B functions as a second BPE level determination unit. The MLM 72 functions as a BPE symmetry determination unit.

The BPE determination processing unit 70A determines the BPE level of the left breast M by inputting the first difference image RC1 to the MLM 71A. The MLM 71A outputs a determination result RB1 of the BPE level for the left breast M. The BPE determination processing unit 70A determines the BPE level of the right breast M by inputting the second difference image RC2 to the MLM 71B. The MLM 71B outputs a determination result RB2 of the BPE level for the right breast M. It should be noted that the BPE level for the left breast M corresponds to a "first enhancement level" according to the present disclosed technology. The BPE level for the right breast M corresponds to a "second enhancement level" according to the present disclosed technology.

The MLM 71A and the MLM 71B are the same model configured by a CNN. It should be noted that the same model refers to a machine learned model having the same configuration, which is obtained by performing machine learning using the same training data.

The BPE determination processing unit 70A acquires one feature map F1B generated by performing the convolution processing via the MLM 71A, and acquires one feature map F2B generated by performing the convolution processing via the MLM 71B. The BPE determination processing unit 70A combines the feature map F1B and the feature map F2B and inputs the combined feature map F1B and feature map F2B, to the MLM 72 to determine the BPE symmetry for the left and right breasts M. It should be noted that the BPE determination processing unit 70A may combine the feature map F1B and the feature map F2B in the channel direction, or may combine the feature map F1B and the feature map F2B in the row direction or the column direction (that is, the X direction or the Y direction).

The MLM 72 is configured by, for example, a CNN, similarly to the MLM 71A and the MLM 71B. The determination result of the BPE symmetry is classified into two classes of "symmetric" and "asymmetric". The MLM 72 outputs a class to which the BPE symmetry of the left and right breasts M belongs, as a determination result RB3.

The lesion candidate region extraction processing unit 53 inputs the determination results RB1 and RB3, which are output from the BPE determination processing unit 70A, to the MLM 61A, and inputs the determination results RB2 and RB3, which are output from the BPE determination processing unit 70A, to the MLM 61B. In the present modification example, the lesion candidate region extraction processing unit 53 adjusts a parameter, such as a threshold value, for determining a condition under which the MLM 61A extracts the lesion candidate region CA, based on the determination results RB1 and RB3. In addition, the lesion candidate region extraction processing unit 53 adjusts a parameter, such as a threshold value, for determining a condition under which the MLM 61B extracts the lesion candidate region CA, based on the determination results RB2 and RB3.

For example, each of the MLM 61A and the MLM 61B includes a classifier that extracts the ROI as the lesion candidate region CA in a case in which the score calculated based on the feature value extracted from the ROI is equal to or greater than a threshold value. The lesion candidate region extraction processing unit 53 changes the threshold value of the classifier included in the MLM 61A in accordance with the BPE level represented by the determination result RB 1 and the BPE symmetry represented by the determination result RB3. In addition, the lesion candidate region extraction processing unit 53 changes the threshold value of the classifier included in the MLM 61B in accordance with the BPE level represented by the determination result RB2 and the BPE symmetry represented by the determination result RB3.

For example, in a case in which the BPE level is high, there is a possibility that the lesion is overlooked because the lesion is buried in the background mammary gland as described above, so that the lesion candidate region extraction processing unit 53 extracts a large number of lesion candidate regions CA by decreasing the threshold value of the classifier. In addition, even in a case in which the BPE symmetry is asymmetric, the lesion candidate region extraction processing unit 53 extracts a large number of lesion candidate regions CA by decreasing the threshold value of the classifier.

According to the present modification example, it is possible to perform the lesion candidate region extraction processing with high robustness against the variation in the BPE level and the BPE symmetry, and the support of the interpretation of the image generated by the contrast-enhanced imaging is further improved.

It should be noted that the lesion candidate region extraction processing unit 53 may change the number of lesion candidate regions CA output by the classifier in accordance with the BPE level and the BPE symmetry, instead of the threshold value of the classifier. In this case, the lesion candidate region extraction processing unit 53 increases the number of lesion candidate regions CA output by the classifier as the BPE level is higher. In addition, in a case in which the BPE symmetry is asymmetric, the lesion candidate region extraction processing unit 53 increases the number of lesion candidate regions CA output by the classifier, as compared with a case in which the BPE symmetry is symmetric.

### [Machine Learning Processing]

Next, an example of the machine learning processing for generating the MLM 61 that functions as the lesion candidate region extraction unit will be described.

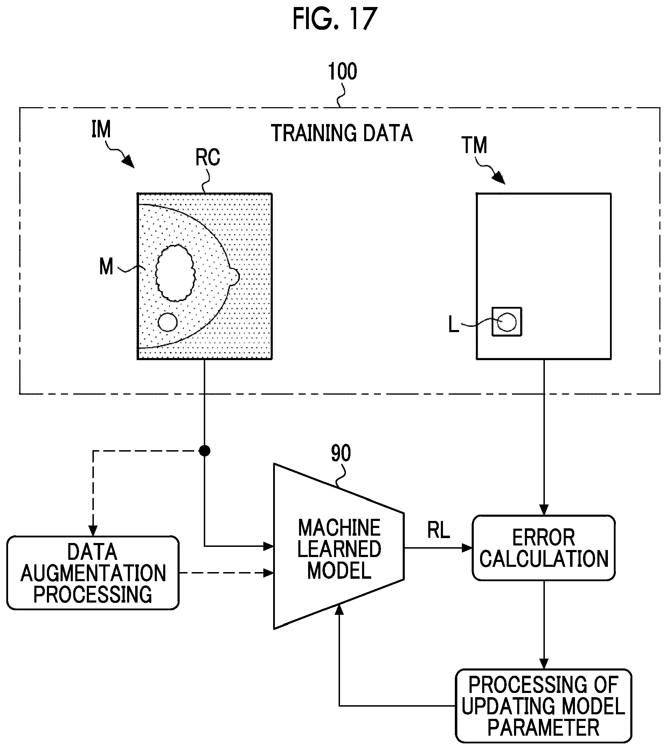

Fig. 17 conceptually shows an example of the machine learning processing of a machine learning model 90. As shown in Fig. 17, the MLM 61 is generated by training the machine learning model 90 through the machine learning using training data 100 in the training phase. For example, the training data 100 is composed of a combination of an input image IM and ground-truth data TM. A large number of input images IM and the ground-truth data TM are used in processing of training the machine learning model 90. For example, the input image IM is the difference image RC. The ground-truth data TM is information representing a region of a true lesion L.

The machine learning model 90 is trained through the machine learning by using, for example, an error back propagation method. In the training phase, an error calculation between an extraction result of the lesion candidate region CA obtained by inputting the input image IM to the machine learning model 90 and the ground-truth data TM, and processing of updating a model parameter of the machine learning model 90 based on a result of the error calculation are repeatedly performed. The model parameter includes the weight of the filter and the like.

Further, in the training phase, data augmentation processing is performed. In the data augmentation processing, an augmented image AM in which a contrast between the region including the lesion L (hereinafter, referred to as a lesion region) and other regions (hereinafter, referred to as a non-lesion region) is changed is generated based on the input image IM. Then, the machine learning of the machine learning model 90 is performed by using the augmented image AM generated by the data augmentation processing and the ground-truth data TM.

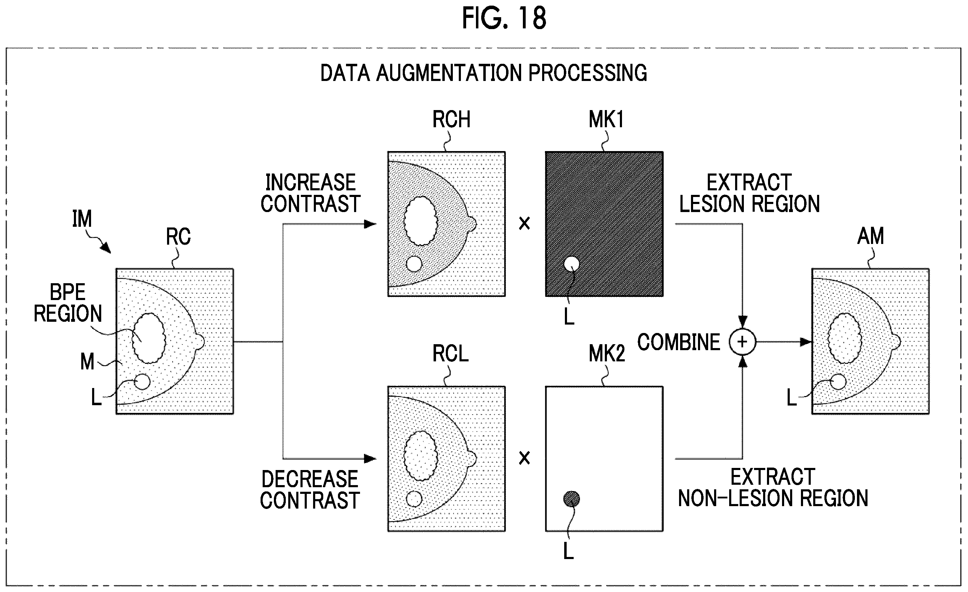

Fig. 18 conceptually shows the data augmentation processing. In the data augmentation processing, for example, a high-contrast image RCH obtained by increasing the contrast of the difference image RC and a low-contrast image RCL obtained by decreasing the contrast of the difference image RC are created. Next, the lesion region is extracted from the high-contrast image RCH by applying mask information MK1 for specifying the lesion region to the high-contrast image RCH. In addition, the non-lesion region is extracted from the low-contrast image RCL by applying mask information MK2 for specifying the non-lesion region to the low-contrast image RCL. The extracted lesion region and non-lesion region are combined to generate the augmented image AM in which the contrast between the lesion region and the non-lesion region is changed.

In the contrast-enhanced imaging, after the contrast agent is injected into the breast M, the contrast between the lesion region and the non-lesion region is changed. For example, since the contrast agent is more likely to be washed out in the BPE region, which is the non-lesion region, than in the lesion region, the contrast between the lesion and the BPE region is changed in accordance with the elapsed time after the contrast agent injection. That is, the contrast between the lesion region and the non-lesion region is changed in accordance with an imaging timing after the contrast agent is injected. Therefore, by performing the above-described data augmentation processing, the machine learning model 90 having high robustness against the variation in the imaging timing can be generated.

In order to increase the robustness, it is preferable to train the machine learning model 90 by generating a plurality of augmented images AM having different contrasts between the lesion region and the non-lesion region from one input image IM.

The machine learning model 90 that has been trained through the machine learning in the training phase is stored in the storage unit 42 as the MLM 61. It should be noted that the machine learning of the machine learning model 90 may be performed by the information processing apparatus 12 or may be performed by an external apparatus.

It should be noted that the MLM 61 may be generated by training the machine learning model 90 through the machine learning using only the low-energy image LE, and then retraining the machine learning model 90 using the difference image RC and the low-energy image LE.

The MLM 62 that functions as the lesion determination unit is generated by the same machine learning processing. Further, the MLMs 71, 71A, and 71B functioning as the BPE level determination unit and the MLM 72 functioning as the BPE symmetry determination unit are generated by the same machine learning processing.

### [Second Embodiment]

Hereinafter, the second embodiment will be described. In the above-described embodiment, the mammography apparatus 10 irradiates the breast M with the radiation R at one angle to acquire the radiation image. In the present embodiment, the mammography apparatus 10 enables tomosynthesis imaging that acquires the series of a plurality of radiation images by irradiating the breast M with the radiation at a plurality of angles.

In the present embodiment, the mammography apparatus 10 acquires the low-energy image LE and the high-energy image HE by irradiating the breast M in which the contrast agent is injected, with the radiation R having different energies for each angle. That is, a low-energy image group LEG consisting of a plurality of low-energy images LE and a high-energy image group HEG consisting of a plurality of high-energy images HE are generated by the tomosynthesis imaging.

In the present embodiment, the difference image generation unit 52 generates the difference image RC representing the difference between the low-energy image LE and the high-energy image HE for each angle. Therefore, a difference image group RCG consisting of a plurality of difference images RC is generated.

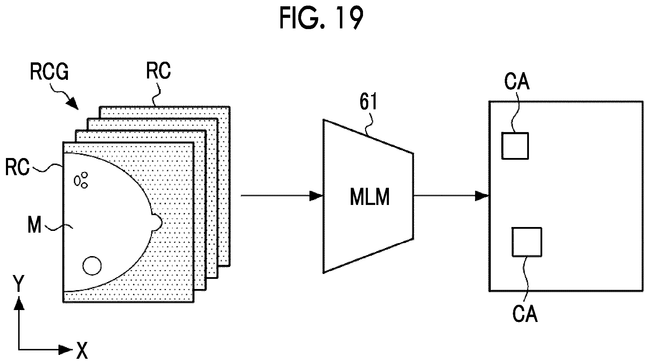

Fig. 19 schematically shows a lesion candidate region extraction processing via the lesion candidate region extraction processing unit 53 according to the second embodiment. In the present embodiment, the lesion candidate region extraction processing unit 53 extracts the lesion candidate region CA by inputting the difference image group RCG to the MLM 61. For the lesion candidate region extraction processing according to the second embodiment, the same modification as in the first modification example, the third modification example, the fourth modification example, or the fifth modification example can be made.

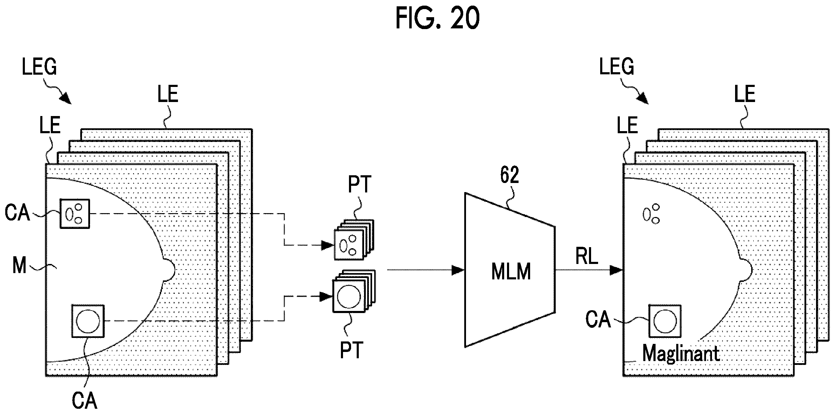

Fig. 20 schematically shows lesion determination processing via the lesion determination processing unit 54 according to the second embodiment. In the present embodiment, the lesion determination processing unit 54 performs the lesion determination by cutting out a voxel corresponding to the lesion candidate region CA from the low-energy image group LEG as a three-dimensional patch image PT and inputting the cutout patch image PT to the MLM 62.

It should be noted that the lesion determination processing unit 54 may cut out the patch image PT from the high-energy image group HEG and input the cutout patch image PT to the MLM 62, instead of the low-energy image group LEG. In addition, the lesion determination processing unit 54 may cut out respective regions corresponding to the same lesion candidate region CA from the low-energy image group LEG and the high-energy image group HEG, as the patch images PT, and input the patch images PT to the MLM 62. For the lesion determination processing according to the second embodiment, the same modification as in the second modification example can be made.

In the present embodiment, the low-energy image LE and the high-energy image HE are acquired by irradiating the breast M with the radiation R having different energies for each angle, so that the misregistration of the breast M between the low-energy image LE and the high-energy image HE acquired at the same angle is suppressed. As a result, the difference image group RCG can be generated with high reliability, and the lesion candidate region CA can be accurately extracted.

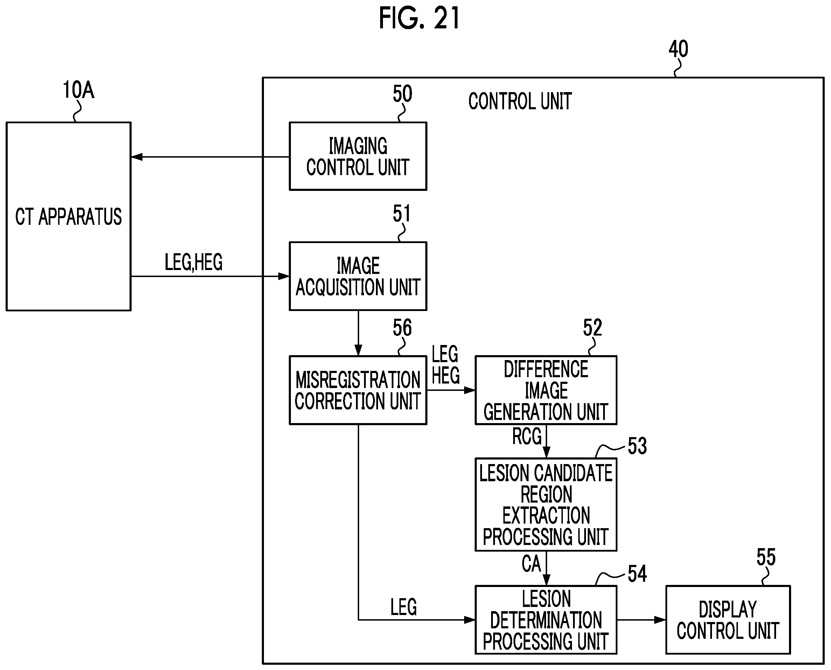

As shown in Fig. 21, as a modification example of the second embodiment, instead of the mammography apparatus 10, a computed tomography (CT) apparatus 10A can be used. The CT apparatus 10A captures a plurality of radiation images while rotating a pair of radiation sources and radiation detectors around the subject in which the contrast agent is injected. The CT apparatus 10A according to the present embodiment enables so-called dual-energy CT that performs the irradiation of the subject with the radiation having the first energy and the irradiation of the subject with the radiation having the second energy higher than the first energy. For example, 360° scanning around the subject is performed twice with the first energy and the second energy. As a result, as in a case of the tomosynthesis imaging, the low-energy image group LEG and the high-energy image group HEG are generated.

In the dual-energy CT, the energy of the radiation is changed for each scanning, and thus it is considered that an amount of misregistration of the subject between the low-energy image LE and the high-energy image HE acquired at the same angle is larger than that in a case of the tomosynthesis imaging. Therefore, as shown in Fig. 21, it is preferable that a misregistration correction unit 56 is provided in the latter part of the image acquisition unit 51. The misregistration correction unit 56 corrects the misregistration of the subject between the low-energy image group LEG and the high-energy image group HEG. The misregistration correction unit 56 supplies the low-energy image group LEG and the high-energy image group HEG in which the misregistration is corrected to the difference image generation unit 52, and supplies the low-energy image group LEG to the lesion determination processing unit 54. Other kinds of processing are the same as in the first embodiment.

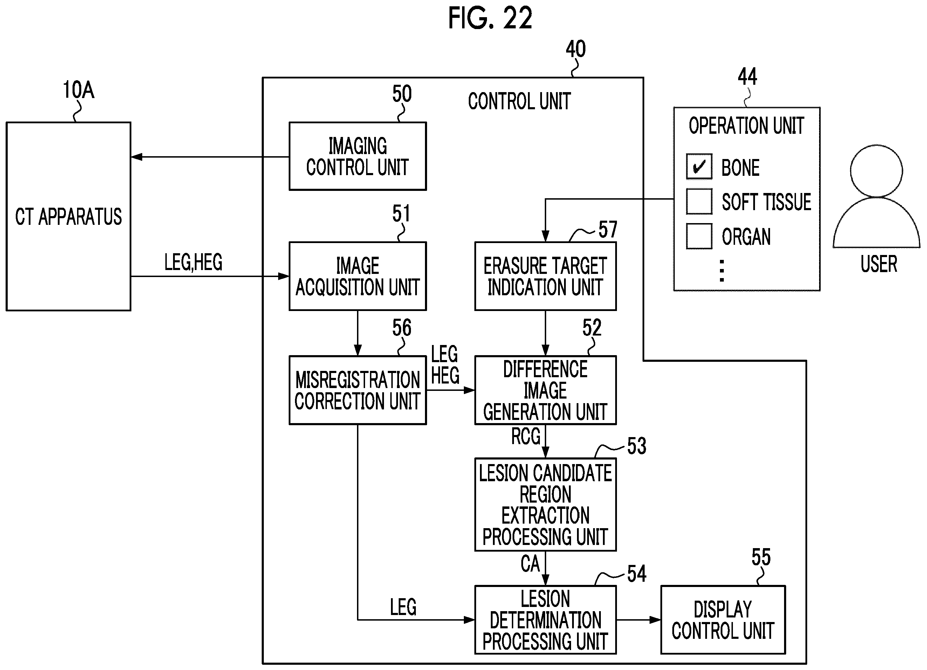

In addition, in the mammography, since the breast is the subject, almost all normal tissues are erased by performing energy subtraction to erase the mammary gland structure, but, in the CT, since a human body or the like is a subject, normal tissues having different compositions are present. Therefore, as shown in Fig. 22, it is preferable to provide an erasure target indication unit 57 that indicates a composition as an erasure target with respect to the difference image generation unit 52.

In the present embodiment, the user can designate the composition as the erasure target by using the operation unit 44. The composition that can be designated as the erasure target is, for example, a bone, a soft tissue, an organ, or the like. The erasure target indication unit 57 acquires information on the erasure target designated by using the operation unit 44, and indicates the erasure target with respect to the difference image generation unit 52. The difference image generation unit 52 erases the composition as the erasure target from the difference image RC by changing the first weight coefficient multiplied by the low-energy image LE and the second weight coefficient multiplied by the high-energy image HE, depending on the erasure target.

Further, instead of the CT apparatus 10A, a magnetic resonance imaging (MRI) apparatus can also be used. The CT apparatus 10A images a moisture content in cells, tissues, and the like in the subject by using the magnetic field. In the MRI, as in the CT, it is possible to perform the energy subtraction by using the contrast agent. In this case, at the high magnetic field, the subject is irradiated with the electromagnetic waves having first energy and irradiated with the electromagnetic waves having second energy higher than the first energy, and the low-energy image group LEG and the high-energy image group HEG are generated.

### [Other Modification Examples]

The control unit 40 may accumulate information related to the contrast-enhanced imaging, information on the person under an examination on which the contrast-enhanced imaging is performed, and the like in the storage unit 42 or the like as data. For example, the control unit 40 may accumulate information such as an injection start time point of the contrast agent into the breast M, an imaging time point of the contrast-enhanced imaging (or an elapsed time from the start of the injection of the contrast agent to the imaging time point), a thickness of the breast M, imaging conditions (a tube voltage and the like), and other patient information (age, menstrual cycle, presence or absence of menopause, and the like) in the storage unit 42 or the like.

In the above-described embodiment and respective modification examples, the lesion determination processing unit 54 determines whether the lesion candidate included in the lesion candidate region CA is benign or malignant, but may perform a category determination, a finding determination (determination of a tumor, calcification, a construction disorder, or the like), and the like.

In addition, in each of the above-described embodiments and each of the above-described modification examples, as a hardware structure of a processing unit that executes various kinds of processing, such as the imaging control unit 50, the image acquisition unit 51, the difference image generation unit 52, the lesion candidate region extraction processing unit 53, the lesion determination processing unit 54, the display control unit 55, the misregistration correction unit 56, and the erasure target indication unit 57, various processors shown later can be used.

The various processors include a graphics processing unit (GPU) as well as a CPU. Further, the various processors include, in addition to a general-purpose processor which executes software (program) and functions as various processing units, such as a CPU, a programmable logic device (PLD) that is a processor whose circuit configuration can be changed after manufacture, such as a field programmable gate array (FPGA), and a dedicated electrical circuit that is a processor having a circuit configuration which is designed for exclusive use in order to execute specific processing, such as an application-specific integrated circuit (ASIC).

One processing unit may be configured by one of the various processors or may be configured by combining two or more processors of the same type or different types (for example, by combining a plurality of FPGAs or combining a CPU and an FPGA). Further, a plurality of the processing units may be configured by one processor.

A first example of the configuration in which the plurality of processing units are configured by one processor is a form in which one processor is configured by combining one or more CPUs and the software and this processor functions as the plurality of processing units, as represented by computers such as a client and a server. A second example is a form of using a processor that implements the function of the entire system including the plurality of processing units via one integrated circuit (IC) chip, as represented by a system on a chip (SoC) or the like. In this way, as the hardware structure, the various processing units are configured by using one or more of the various processors described above.

Further, the hardware structure of the various processors is, more specifically, an electric circuit (circuitry) in which circuit elements such as semiconductor elements are combined.

In addition, in the above-described embodiment and respective modification examples, the aspect has been described in which the program 42A is stored in the storage unit 42 in advance, but the present disclosure is not limited to this. The program 42A may be provided in a form of being recorded in a non-transitory recording medium such as a compact disc read only memory (CD-ROM), a digital versatile disc read only memory (DVD-ROM), or a universal serial bus (USB) memory. Further, the program 42A may be downloaded from an external apparatus via a network.

The above-described embodiment and respective modification examples can be combined as appropriate as long as there is no contradiction.

The above-described contents and the above-shown contents are detailed descriptions of portions related to the present disclosed technology and are merely examples of the present disclosed technology. For example, the description of the configuration, the function, the operation, and the effect are the description of examples of the configuration, the function, the operation, and the effect of the parts according to the present disclosed technology. Therefore, it goes without saying that unnecessary parts may be deleted, new elements may be added, or replacements may be made with respect to the above-described contents and the above-shown contents within a range that does not deviate from the scope of the present disclosed technology. Further, the description of, for example, common technical knowledge that does not need to be particularly described to enable the implementation of the present disclosed technology is omitted in the above-described contents and the above-shown contents in order to avoid confusion and to facilitate the understanding of the portions related to the present disclosed technology.

## Claims

1. An information processing apparatus (12) comprising:
at least one processor,
wherein the processor is configured to:
generate a difference image (S21) representing a difference between a low-energy image captured by irradiating a subject, in which a contrast agent is injected, with electromagnetic waves having first energy and a high-energy image captured by irradiating the subject with electromagnetic waves having second energy higher than the first energy;
extract a lesion candidate region (S22) including a lesion candidate from the difference image; and wherein the information processing apparatus is **characterized in that** the processor is further configured to
cut out a region corresponding to the lesion candidate region from at least any one of the low-energy image or the high-energy image, as a patch image; and
determine whether or not the lesion candidate is a lesion based on the patch image;
wherein the processor is configured to generate the difference image by subtracting an image obtained by multiplying the low-energy image by a first weight coefficient from an image obtained by multiplying the high-energy image by a second weight coefficient for each corresponding pixel.

2. The information processing apparatus (12) according to claim 1,
wherein the processor is configured to:
determine whether or not the lesion candidate is the lesion by inputting the patch image to a machine learned model.

3. The information processing apparatus (12) according to claim 2,
wherein the processor is configured to:
cut out respective regions corresponding to the same lesion candidate region from at least any one of the low-energy image or the high-energy image and the difference image, as the patch images, and input the cutout regions to the machine learned model.

4. The information processing apparatus (12) according to claim 2,
wherein the subject is a breast, and
the processor is configured to:
determine an enhancement level of background mammary gland parenchyma of the breast based on the difference image and adjust a parameter for determining a condition under which the machine learned model extracts the lesion candidate region, based on the enhancement level.

5. The information processing apparatus (12) according to claim 4,
wherein the electromagnetic waves are radiation.

6. The information processing apparatus (12) according to claim 1,
wherein the subject is left and right breasts,
the low-energy image includes a first low-energy image and a second low-energy image that are captured by irradiating each of the left and right breasts with radiation having the first energy,
the high-energy image includes a first high-energy image and a second high-energy image that are captured by irradiating each of the left and right breasts with radiation having the second energy, and
the difference image includes a first difference image representing a difference between the first low-energy image and the first high-energy image and a second difference image representing a difference between the second low-energy image and the second high-energy image.

7. The information processing apparatus (12) according to claim 6,
wherein the processor is configured to:
extract the lesion candidate region from the first difference image by inputting the first difference image to a first machine learned model; and
extract the lesion candidate region from the second difference image by inputting the second difference image to a second machine learned model.

8. The information processing apparatus (12) according to claim 7,
wherein the first machine learned model includes a first pre-stage operation block and a first post-stage operation block,
the second machine learned model includes a second pre-stage operation block and a second post-stage operation block, and
the processor is configured to:
extract a first feature value by inputting the first difference image to the first pre-stage operation block;
extract a second feature value by inputting the second difference image to the second pre-stage operation block;
extract the lesion candidate region from the first difference image by combining the second feature value with the first feature value and inputting the combined feature value to the first post-stage operation block; and
extract the lesion candidate region from the second difference image by combining the first feature value with the second feature value and inputting the combined feature value to the second post-stage operation block.

9. The information processing apparatus (12) according to claim 8,
wherein the processor is configured to:
combine the first feature value and the second feature value in a channel direction.

10. The information processing apparatus (12) according to claim 8,
wherein the processor is configured to:
combine the first feature value and the second feature value via a cross attention mechanism that generates a weight map representing a degree of relevance between the first feature value and the second feature value and that performs weighting on the first feature value and the second feature value based on the generated weight map.

11. The information processing apparatus (12) according to claim 7,
wherein the processor is configured to:
determine a first enhancement level of background mammary gland parenchyma based on the first difference image;
determine a second enhancement level of the background mammary gland parenchyma based on the second difference image;
determine symmetry of enhancement regions of the background mammary gland parenchyma related to the left and right breasts based on the first difference image and the second difference image;
adjust a parameter for determining a condition under which the first machine learned model extracts the lesion candidate region, based on the first enhancement level and the symmetry; and
adjust a parameter for determining a condition under which the second machine learned model extracts the lesion candidate region, based on the second enhancement level and the symmetry.

12. The information processing apparatus (12) according to claim 2,
wherein the machine learned model is generated by training a machine learning model using training data including an input image and ground-truth data and an augmented image in which a contrast between a lesion region and a non-lesion region in the input image is changed.

13. An information processing method comprising:
generating a difference image (S21) representing a difference between a low-energy image captured by irradiating a subject, in which a contrast agent is injected, with electromagnetic waves having first energy and a high-energy image captured by irradiating the subject with electromagnetic waves having second energy higher than the first energy;
extracting a lesion candidate region (S22) including a lesion candidate from the difference image; wherein the information processing method is **characterized in that** it further comprises
cutting out a region corresponding to the lesion candidate region from at least any one of the low-energy image or the high-energy image, as a patch image; and
determining whether or not the lesion candidate is a lesion based on the patch image;
wherein the method comprises generating the difference image by subtracting an image obtained by multiplying the low-energy image by a first weight coefficient from an image obtained by multiplying the high-energy image by a second weight coefficient for each corresponding pixel.

14. A computer program comprising a process with instructions which, when the program is executed by a computer, cause the computer to carry out:
generating a difference image (S21) representing a difference between a low-energy image captured by irradiating a subject, in which a contrast agent is injected, with electromagnetic waves having first energy and a high-energy image captured by irradiating the subject with electromagnetic waves having second energy higher than the first energy;
extracting a lesion candidate region (S22) including a lesion candidate from the difference image; wherein the computer program is **characterized in that** the process further comprises
cutting out a region corresponding to the lesion candidate region from at least any one of the low-energy image or the high-energy image, as a patch image; and
determining whether or not the lesion candidate is a lesion based on the patch image;
wherein the process comprises generating the difference image by subtracting an image obtained by multiplying the low-energy image by a first weight coefficient from an image obtained by multiplying the high-energy image by a second weight coefficient for each corresponding pixel.

## Patentansprüche

1. Informationsverarbeitungsvorrichtung (12), umfassend:
mindestens einen Prozessor,
wobei der Prozessor so konfiguriert ist, dass er:
ein Differenzbild (S21), das eine Differenz zwischen einem Niedrigenergiebild, das durch Bestrahlen eines Untersuchungsobjekts, in das ein Kontrastmittel injiziert wird, mit elektromagnetischen Wellen, die erste Energie aufweisen, aufgenommen worden ist, und einem Hochenergiebild, das durch Bestrahlen des Untersuchungsobjekts mit elektromagnetischen Wellen, die zweite Energie aufweisen, die höher als die erste Energie ist, aufgenommen worden ist, darstellt, erzeugt;
einen Läsionskandidatenbereich (S22), der einen Läsionskandidaten enthält, aus dem Differenzbild extrahiert; und wobei die Informationsverarbeitungsvorrichtung **dadurch gekennzeichnet ist, dass** der Prozessor ferner so konfiguriert ist, dass er
einen Bereich, der dem Läsionskandidatenbereich entspricht, aus mindestens einem von dem Niedrigenergiebild und dem Hochenergiebild als ein Patch-Bild ausschneidet; und
auf der Grundlage des Patch-Bildes bestimmt, ob der Läsionskandidat eine Läsion ist oder nicht;
wobei der Prozessor so konfiguriert ist, dass er das Differenzbild durch Subtrahieren eines Bildes, das durch Multiplizieren des Niedrigenergiebildes mit einem ersten Gewichtskoeffizienten erhalten wird, von einem Bild, das durch Multiplizieren des Hochenergiebildes mit einem zweiten Gewichtskoeffizienten für jedes entsprechende Pixel erhalten wird, erzeugt.

2. Informationsverarbeitungsvorrichtung (12) nach Anspruch 1,
wobei der Prozessor so konfiguriert ist, dass er:
bestimmt, ob der Läsionskandidat die Läsion ist oder nicht, indem er das Patch-Bild in ein maschinell gelerntes Modell eingibt.

3. Informationsverarbeitungsvorrichtung (12) nach Anspruch 2,
wobei der Prozessor so konfiguriert ist, dass er:
jeweilige Bereiche, die dem gleichen Läsionskandidatenbereich entsprechen, aus mindestens einem von dem Niedrigenergiebild und dem Hochenergiebild und aus dem Differenzbild als die Patch-Bilder ausschneidet und die ausgeschnittenen Bereiche in das maschinell gelernte Modell eingibt.

4. Informationsverarbeitungsvorrichtung (12) nach Anspruch 2,
wobei das Untersuchungsobjekt eine Brust ist, und
der Prozessor so konfiguriert ist, dass er:
auf der Grundlage des Differenzbildes ein Verstärkungsniveau von Hintergrund - Brustdrüsenparenchym der Brust bestimmt und einen Parameter zum Bestimmen einer Bedingung, unter der das maschinell gelernte Modell den Läsionskandidatenbereich extrahiert, auf der Grundlage des Verstärkungsniveaus anpasst.

5. Informationsverarbeitungsvorrichtung (12) nach Anspruch 4,
wobei die elektromagnetischen Wellen Strahlung sind.

6. Informationsverarbeitungsvorrichtung (12) nach Anspruch 1,
wobei das Untersuchungsobjekt eine linke und eine rechte Brust sind,
das Niedrigenergiebild ein erstes Niedrigenergiebild und ein zweites Niedrigenergiebild, die durch Bestrahlen jeweils der linken und rechten Brust mit Strahlung, die die erste Energie aufweist, aufgenommen werden, enthält,
das Hochenergiebild ein erstes Hochenergiebild und ein zweites Hochenergiebild, die durch Bestrahlen jeweils der linken und rechten Brust mit Strahlung, die die zweite Energie aufweist, aufgenommen werden, enthält, und
das Differenzbild ein erstes Differenzbild, das eine Differenz zwischen dem ersten Niedrigenergiebild und dem ersten Hochenergiebild darstellt, und ein zweites Differenzbild, das eine Differenz zwischen dem zweiten Niedrigenergiebild und dem zweiten Hochenergiebild darstellt, enthält.

7. Informationsverarbeitungsvorrichtung (12) nach Anspruch 6,
wobei der Prozessor so konfiguriert ist, dass er:
den Läsionskandidatenbereich aus dem ersten Differenzbild durch Eingeben des ersten Differenzbildes in ein erstes maschinell gelerntes Modell extrahiert; und
den Läsionskandidatenbereich aus dem zweiten Differenzbild durch Eingeben des zweiten Differenzbildes in ein zweites maschinell gelerntes Modell extrahiert.

8. Informationsverarbeitungsvorrichtung (12) nach Anspruch 7,
wobei das erste maschinell gelernte Modell einen ersten Vorstufen-Operationsblock und einen ersten Nachstufen-Operationsblock enthält,
das zweite maschinell gelernte Modell einen zweiten Vorstufen-Operationsblock und einen zweiten Nachstufen-Operationsblock enthält, und
der Prozessor so konfiguriert ist, dass er:
einen ersten Merkmalswert durch Eingeben des ersten Differenzbildes in den ersten Vorstufen-Operationsblock extrahiert;
einen zweiten Merkmalswert durch Eingeben des zweiten Differenzbildes in den zweiten Vorstufen-Operationsblock extrahiert;
den Läsionskandidatenbereich aus dem ersten Differenzbild durch Kombinieren des zweiten Merkmalswerts mit dem ersten Merkmalswert und Eingeben des kombinierten Merkmalswerts in den ersten Nachstufen-Operationsblock extrahiert; und
den Läsionskandidatenbereich aus dem zweiten Differenzbild durch Kombinieren des ersten Merkmalswerts mit dem zweiten Merkmalswert und Eingeben des kombinierten Merkmalswerts in den zweiten Nachstufen-Operationsblock extrahiert.

9. Informationsverarbeitungsvorrichtung (12) nach Anspruch 8,
wobei der Prozessor so konfiguriert ist, dass er:
den ersten Merkmalswert und den zweiten Merkmalswert in einer Kanalrichtung kombiniert.

10. Informationsverarbeitungsvorrichtung (12) nach Anspruch 8,
wobei der Prozessor so konfiguriert ist, dass er:
den ersten Merkmalswert und den zweiten Merkmalswert via einen Cross-Attention-Mechanismus, der eine Gewichtskarte, die einen Relevanzgrad zwischen dem ersten Merkmalswert und dem zweiten Merkmalswert darstellt, erzeugt und der auf der Grundlage der erzeugten Gewichtskarte eine Gewichtung an dem ersten Merkmalswert und dem zweiten Merkmalswert durchführt, kombiniert.

11. Informationsverarbeitungsvorrichtung (12) nach Anspruch 7,
wobei der Prozessor so konfiguriert ist, dass er:
ein erstes Verstärkungsniveau von Hintergrund-Brustdrüsenparenchym auf der Grundlage des ersten Differenzbildes bestimmt;
ein zweites Verstärkungsniveau des Hintergrund-Brustdrüsenparenchyms auf der Grundlage des zweiten Differenzbildes bestimmt;
Symmetrie von Verstärkungsbereichen des Hintergrund-Brustdrüsenparenchyms, die sich auf die linke und rechte Brüste beziehen, auf der Grundlage des ersten Differenzbildes und des zweiten Differenzbildes bestimmt;
einen Parameter zum Bestimmen einer Bedingung, unter der das erste maschinell gelernte Modell den Läsionskandidatenbereich extrahiert, auf der Grundlage des ersten Verstärkungsniveaus und der Symmetrie anpasst; und
einen Parameter zum Bestimmen einer Bedingung, unter der das zweite maschinell gelernte Modell den Läsionskandidatenbereich extrahiert, auf der Grundlage des zweiten Verstärkungsniveaus und der Symmetrie anpasst.

12. Informationsverarbeitungsvorrichtung (12) nach Anspruch 2,
wobei das maschinell gelernte Modell durch Trainieren eines Modells für maschinelles Lernen unter Verwendung von Trainingsdaten, die ein Eingabebild und Ground-Truth-Daten und ein erweitertes Bild, in dem ein Kontrast zwischen einem Läsionsbereich und einem Nichtläsionsbereich in dem Eingabebild geändert ist, enthalten, erzeugt wird.

13. Informationsverarbeitungsverfahren, umfassend:
Erzeugen eines Differenzbildes (S21), das eine Differenz zwischen einem Niedrigenergiebild, das durch Bestrahlen eines Untersuchungsobjekts, in das ein Kontrastmittel injiziert wird, mit elektromagnetischen Wellen, die erste Energie aufweisen, aufgenommen worden ist, und einem Hochenergiebild, das durch Bestrahlen des Untersuchungsobjekts mit elektromagnetischen Wellen, die zweite Energie aufweisen, die höher als die erste Energie ist, aufgenommen worden ist, darstellt;
Extrahieren eines Läsionskandidatenbereichs (S22), der einen Läsionskandidaten enthält, aus dem Differenzbild; wobei das Informationsverarbeitungsverfahren **dadurch gekennzeichnet ist, dass** es ferner umfasst:
Ausschneiden eines Bereichs, der dem Läsionskandidatenbereich entspricht, aus mindestens einem von dem Niedrigenergiebild und dem Hochenergiebild als ein Patch-Bild; und
Bestimmen, ob der Läsionskandidat eine Läsion ist oder nicht, auf der Grundlage des Patch-Bildes;
wobei das Verfahren Erzeugen des Differenzbildes durch Subtrahieren eines Bildes,
das durch Multiplizieren des Niedrigenergiebildes mit einem ersten Gewichtskoeffizienten erhalten wird, von einem Bild, das durch Multiplizieren des Hochenergiebildes mit einem zweiten Gewichtskoeffizienten für jedes entsprechende Pixel erhalten wird, umfasst.

14. Computerprogramm, das einen Prozess mit Anweisungen umfasst, die, wenn das Programm von einem Computer ausgeführt wird, den Computer veranlassen, Folgendes durchzuführen:
Erzeugen eines Differenzbildes (S21), das eine Differenz zwischen einem Niedrigenergiebild, das durch Bestrahlen eines Untersuchungsobjekts, in das ein Kontrastmittel injiziert wird, mit elektromagnetischen Wellen, die erste Energie aufweisen, aufgenommen worden ist, und einem Hochenergiebild, das durch Bestrahlen des Untersuchungsobjekts mit elektromagnetischen Wellen, die zweite Energie aufweisen, die höher als die erste Energie ist, aufgenommen worden ist, darstellt;
Extrahieren eines Läsionskandidatenbereichs (S22), der einen Läsionskandidaten enthält, aus dem Differenzbild; wobei das Computerprogramm **dadurch gekennzeichnet ist, dass** der Prozess ferner umfasst:
Ausschneiden eines Bereichs, der dem Läsionskandidatenbereich entspricht, aus mindestens einem von dem Niedrigenergiebild und dem Hochenergiebild als ein Patch-Bild; und
Bestimmen, ob der Läsionskandidat eine Läsion ist oder nicht, auf der Grundlage des Patch-Bildes;
wobei der Prozess Erzeugen des Differenzbildes durch Subtrahieren eines Bildes, das durch Multiplizieren des Niedrigenergiebildes mit einem ersten Gewichtskoeffizienten erhalten wird, von einem Bild, das durch Multiplizieren des Hochenergiebildes mit einem zweiten Gewichtskoeffizienten für jedes entsprechende Pixel erhalten wird, umfasst.

## Revendications

1. Appareil de traitement d'informations (12) comprenant :
au moins un processeur,
dans lequel le processeur est configuré pour :
générer une image de différence (S21) représentant une différence entre une image à faible énergie capturée en irradiant un sujet, dans lequel un agent de contraste est injecté, avec des ondes électromagnétiques ayant une première énergie et une image à haute énergie capturée en irradiant le sujet avec des ondes électromagnétiques ayant une deuxième énergie supérieure à la première énergie ;
extraire une région de lésion candidate (S22) incluant une lésion candidate à partir de l'image de différence ; et dans lequel l'appareil de traitement d'informations est **caractérisé en ce que** le processeur est en outre configuré pour
découper une région correspondant à la région de lésion candidate à partir d'au moins l'une quelconque de l'image à faible énergie ou de l'image à haute énergie, en tant qu'image de patch ; et
déterminer si la lésion candidate est ou non une lésion sur la base de l'image de patch ;
dans lequel le processeur est configuré pour générer l'image de différence en soustrayant, pour chaque pixel correspondant, une image obtenue en multipliant l'image à faible énergie par un premier coefficient de pondération d'une image obtenue en multipliant l'image à haute énergie par un deuxième coefficient de pondération.

2. Appareil de traitement d'informations (12) selon la revendication 1,
dans lequel le processeur est configuré pour :
déterminer si la lésion candidate est ou non la lésion en entrant l'image de patch dans un modèle appris par machine.

3. Appareil de traitement d'informations (12) selon la revendication 2,
dans lequel le processeur est configuré pour :
découper des régions respectives correspondant à la même région de lésion candidate à partir d'au moins l'une quelconque de l'image à faible énergie ou de l'image à haute énergie et de l'image de différence, en tant qu'images de patch, et entrer les régions découpées dans le modèle appris par machine.

4. Appareil de traitement d'informations (12) selon la revendication 2,
dans lequel le sujet est un sein, et
le processeur est configuré pour :
déterminer un niveau de rehaussement du parenchyme glandulaire mammaire de fond du sein sur la base de l'image de différence et ajuster un paramètre pour déterminer une condition dans laquelle le modèle appris par machine extrait la région de lésion candidate, sur la base du niveau de rehaussement.

5. Appareil de traitement d'informations (12) selon la revendication 4,
dans lequel les ondes électromagnétiques sont un rayonnement.

6. Appareil de traitement d'informations (12) selon la revendication 1,
dans lequel le sujet est les seins gauche et droit,
l'image à faible énergie inclut une première image à faible énergie et une deuxième image à faible énergie qui sont capturées en irradiant chacun des seins gauche et droit avec un rayonnement ayant la première énergie,
l'image à haute énergie inclut une première image à haute énergie et une deuxième image à haute énergie qui sont capturées en irradiant chacun des seins gauche et droit avec un rayonnement ayant la deuxième énergie, et
l'image de différence inclut une première image de différence représentant une différence entre la première image à faible énergie et la première image à haute énergie et une deuxième image de différence représentant une différence entre la deuxième image à faible énergie et la deuxième image à haute énergie.

7. Appareil de traitement d'informations (12) selon la revendication 6,
dans lequel le processeur est configuré pour :
extraire la région de lésion candidate à partir de la première image de différence en entrant la première image de différence dans un premier modèle appris par machine ; et
extraire la région de lésion candidate à partir de la deuxième image de différence en entrant la deuxième image de différence dans un deuxième modèle appris par machine.

8. Appareil de traitement d'informations (12) selon la revendication 7,
dans lequel le premier modèle appris par machine inclut un premier bloc d'opération pré-étage et un premier bloc d'opération post-étage,
le deuxième modèle appris par machine inclut un deuxième bloc d'opération pré-étage et un deuxième bloc d'opération post-étage, et
le processeur est configuré pour :
extraire une première valeur caractéristique en entrant la première image de différence dans le premier bloc d'opération pré-étage ;
extraire une deuxième valeur caractéristique en entrant la deuxième image de différence dans le deuxième bloc d'opération pré-étage ;
extraire la région de lésion candidate à partir de la première image de différence en combinant la deuxième valeur caractéristique avec la première valeur caractéristique et
en entrant la valeur caractéristique combinée dans le premier bloc d'opération post-étage ; et
extraire la région de lésion candidate à partir de la deuxième image de différence en combinant la première valeur caractéristique avec la deuxième valeur caractéristique et en entrant la valeur caractéristique combinée dans le deuxième bloc d'opération post-étage.

9. Appareil de traitement d'informations (12) selon la revendication 8,
dans lequel le processeur est configuré pour :
combiner la première valeur caractéristique et la deuxième valeur caractéristique dans une direction de canal.

10. Appareil de traitement d'informations (12) selon la revendication 8,
dans lequel le processeur est configuré pour :
combiner la première valeur caractéristique et la deuxième valeur caractéristique via un mécanisme d'attention croisée qui génère une carte de pondération représentant un degré de pertinence entre la première valeur caractéristique et la deuxième valeur caractéristique et qui effectue une pondération sur la première valeur caractéristique et la deuxième valeur caractéristique sur la base de la carte de pondération générée.

11. Appareil de traitement d'informations (12) selon la revendication 7,
dans lequel le processeur est configuré pour :
déterminer un premier niveau de rehaussement du parenchyme glandulaire mammaire de fond sur la base de la première image de différence ;
déterminer un deuxième niveau de rehaussement du parenchyme glandulaire mammaire de fond sur la base de la deuxième image de différence ;
déterminer une symétrie de régions de rehaussement du parenchyme glandulaire mammaire de fond liées aux seins gauche et droit sur la base de la première image de différence et de la deuxième image de différence ;
ajuster un paramètre pour déterminer une condition dans laquelle le premier modèle appris par machine extrait la région de lésion candidate, sur la base du premier niveau de rehaussement et de la symétrie ; et
ajuster un paramètre pour déterminer une condition dans laquelle le deuxième modèle appris par machine extrait la région de lésion candidate, sur la base du deuxième niveau de rehaussement et de la symétrie.

12. Appareil de traitement d'informations (12) selon la revendication 2,
dans lequel le modèle appris par machine est généré en entraînant un modèle d'apprentissage automatique en utilisant des données d'entraînement incluant une image d'entrée et des données de vérité terrain et une image augmentée dans laquelle un contraste entre une région de lésion et une région de non-lésion dans l'image d'entrée est modifié.

13. Procédé de traitement d'informations comprenant :
générer une image de différence (S21) représentant une différence entre une image à faible énergie capturée en irradiant un sujet, dans lequel un agent de contraste est injecté, avec des ondes électromagnétiques ayant une première énergie et une image à haute énergie capturée en irradiant le sujet avec des ondes électromagnétiques ayant une deuxième énergie supérieure à la première énergie ;
extraire une région de lésion candidate (S22) incluant une lésion candidate à partir de l'image de différence ; dans lequel le procédé de traitement d'informations est **caractérisé en ce qu'**il comprend en outre
découper une région correspondant à la région de lésion candidate à partir d'au moins l'une quelconque de l'image à faible énergie ou de l'image à haute énergie, en tant qu'image de patch ; et
déterminer si la lésion candidate est ou non une lésion sur la base de l'image de patch ;
dans lequel le procédé comprend la génération de l'image de différence en soustrayant, pour chaque pixel correspondant, une image obtenue en multipliant l'image à faible énergie par un premier coefficient de pondération d'une image obtenue en multipliant l'image à haute énergie par un deuxième coefficient de pondération.

14. Programme informatique comprenant un processus comportant des instructions qui, lorsque le programme est exécuté par un ordinateur, amènent l'ordinateur à effectuer :
générer une image de différence (S21) représentant une différence entre une image à faible énergie capturée en irradiant un sujet, dans lequel un agent de contraste est injecté, avec des ondes électromagnétiques ayant une première énergie et une image à haute énergie capturée en irradiant le sujet avec des ondes électromagnétiques ayant une deuxième énergie supérieure à la première énergie ;
extraire une région de lésion candidate (S22) incluant une lésion candidate à partir de l'image de différence ; dans lequel le programme informatique est **caractérisé en ce que** le processus comprend en outre
découper une région correspondant à la région de lésion candidate à partir d'au moins l'une quelconque de l'image à faible énergie ou de l'image à haute énergie, en tant qu'image de patch ; et
déterminer si la lésion candidate est ou non une lésion sur la base de l'image de patch ;
dans lequel le processus comprend la génération de l'image de différence en soustrayant, pour chaque pixel correspondant, une image obtenue en multipliant l'image à faible énergie par un premier coefficient de pondération d'une image obtenue en multipliant l'image à haute énergie par un deuxième coefficient de pondération.
